# EUROPÄISCHE PATENTSCHRIFT

(11) **EP 1 395 570 B1**
(45) Veröffentlichungstag und Bekanntmachung des Hinweises auf die Patenterteilung: **10.08.2005**
(21) Anmeldenummer: 02732728.7
(22) Anmeldetag: 17.05.2002
(51) Int. Cl.: C07D 265/24, C07D 498/04, C07D 513/04, A01N 43/86, A01N 43/90

(54) **OXAZIN(THI)ONVERBINDUNGEN ALS FUNGIZIDE**
OXAZIN(THI)ONE COMPOUNDS USED AS FUNGICIDES
COMPOSES D'OXAZIN(THI)ONE UTILISES COMME FONGICIDES

(30) Priorität: 21.05.2001 DE 10124798
(43) Veröffentlichungstag der Anmeldung: 10.03.2004
(73) Patentinhaber: BASF AKTIENGESELLSCHAFT, 67056 Ludwigshafen (DE)
(72) Erfinder: RHEINHEIMER, Joachim, 67063 Ludwigshafen (DE); GYPSER, Andreas, 68159 Mannheim (DE); ROSE, Ingo, 68159 Mannheim (DE); GROTE, Thomas, 67157 Wachenheim (DE); SCHÄFER, Peter, 67308 Ottersheim (DE); SCHIEWECK, Frank, 67258 Hessheim (DE); AMMERMANN, Eberhard, 64646 Heppenheim (DE); SPEAKMAN, John-Bryan, 67273 Bobenheim (DE); STRATHMANN, Siegfried, D-67117 Limburgerhof (DE); LORENZ, Gisela, 67434 Hambach (DE)
(74) Vertreter: Pohl, Michael, Dr.
(86) Internationale Anmeldenummer: PCT/EP2002/005499
(87) Internationale Veröffentlichungsnummer: WO 2002/094797

(56) Entgegenhaltungen:
- WO-A-00/51992
- DE-A- 1 956 043
- DE-A- 2 218 301
- US-A- 3 491 092

## Beschreibung

Die Erfindung betrifft neue Oxazin(thi)onverbindungen und ihre Verwendung zur Bekämpfung von pflanzenpathogenen Pilzen, sowie Mittel zur Bekämpfung von pflanzenpathogenen Pilzen, die eine fungizid wirksame Menge dieser Oxazin(thi)onverbindungen enthalten.

Aus der DE-A 2218301 sind fungizid wirksame Benzo-1-oxa-3-azin-4-one und Benzo-1-thia-3-azin-4-one bekannt, die in der 2-Position eine Trifluormethyliminogruppe aufweisen.

Aus der WO 00/51992 sind fungizid wirksame Azinonverbindungen der Formeln A und B bekannt, 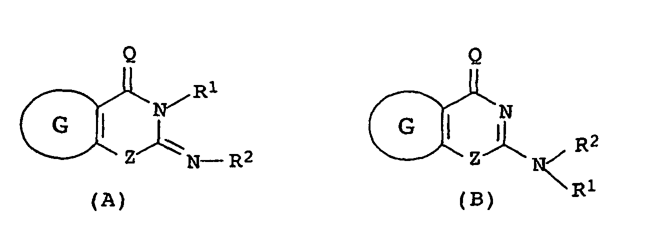 worin Q für Sauerstoff oder Schwefel und Z für Sauerstoff, Schwefel oder eine N-Alkylimin-Gruppe stehen, G einen anellierten Benzolring oder einen anellierten aromatischen Heterocyclus bedeutet und worin R¹ und R² unter anderem für gegebenenfalls substituiertes Alkyl stehen.

Aus US 3,491,092 sind u.a. Verbindungen bekannt, die in der Z-Position einen Hydroxyliminosubstituenten tragen.

Die aus dem Stand der Technik bekannten Fungizide auf der Basis von Oxazinonen lassen teilweise hinsichtlich ihrer Aktivität und/oder Selektivität gegenüber pflanzenpathogenen Pilzen zu wünschen übrig.

Der vorliegenden Erfindung liegt somit die Aufgabe zugrunde, neue Fungizide bereitzustellen, mit denen sich pflanzenpathogene Pilze besser als bisher bekämpfen lassen. Die neuen Fungizide sollen vorteilhafterweise eine hohe Aktivität gegenüber pflanzenpathogenen Pilzen aufweisen.

Diese Aufgabe wird erfindungsgemäß durch die Oxazin(thi)onverbindungen der nachstehend definierten, allgemeinen Formel I gelöst.

Die Erfindung betrifft daher Oxazin(thi)onverbindungen der allgemeinen Formel I: 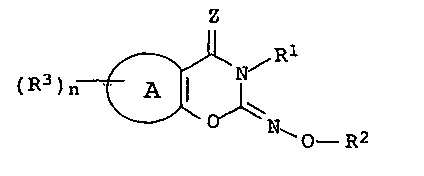 in der die Variablen Z, R¹, R², R³ und n die folgenden Bedeutungen haben:
- Z: Sauerstoff oder Schwefel,
- R¹: Wasserstoff, C₁-C₆-Alkyl, C₂-C₆-Alkenyl, C₂-C₆-Alkinyl, die jeweils gegebenenfalls durch O-R⁴, C(O)O-R⁵, NR⁶R⁷, C(O)NR⁶R⁷, S-R⁸, C₃-C₈-Cycloalkyl, Phenyl oder durch 5- oder 6-gliedriges, gesättigtes oder ungesättigtes Heterocyclyl mit 1 bis 3 Heteroatomen, ausgewählt unter N, O und S, substituiert sind, wobei Phenyl und Heterocyclyl gegebenenfalls ein-, zwei- oder dreifach substituiert sind, C₁-C₆-Halogenalkyl, C₂-C₆-Halogenalkenyl oder C₂-C₆-Halogenalkinyl;
- R²: C₁-C₃-Alkyl, C₁-C₃-Halogenalkyl oder Cyclopropylmethyl;
- R³: Halogen, Cyano, C₁-C₆-Alkyl, C₁-C₆-Halogenalkyl, C₁-C₆-Alkoxy oder C₁-C₆-Halogenalkoxy;
- n: eine Zahl von 0 bis 4;
- A: ein über zwei C-Atome mit dem Oxazin(thi)onring anellierter 5- oder 6-gliedriger Carbocyclus oder ein 5- oder 6-gliedriger Heterocyclus mit 1, 2 oder 3 Heteroatomen, ausgewählt unter N, O und S; und worin
- R⁴,: R⁵ unabhängig voneinander für Wasserstoff, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, Hydroxy-C₁-C₄-alkyl, Hydroxycarbonyl-C₁-C₄-alkyl, C₁-C₄-Alkoxy-C₁-C₄-alkyl oder C₁-C₄-Alkoxycarbonyl-C₁-C₄-alkyl;
- R⁶,: R⁷ unabhängig voneinander für Wasserstoff, C₁-C₆-Alkyl, C₁-C₆-Alkoxy, C₂-C₆-Alkenyl, C₂-C₆-Alkinyl, C₁-C₆-Halogenalkyl, C₁-C₆-Halogenalkoxy, C₂-C₆-Halogenalkenyl, C₂-C₆-Halogenalkinyl, Hydroxy-C₁-C₄-alkyl, Hydroxycarbonyl-C₁-C₄-alkyl, C₁-C₄-Alkoxy-C₁-C₄-alkyl oder C₁-C₄-Alkoxycarbonyl-C₁-C₄-alkyl stehen;
- R⁶: und R⁷ gemeinsam mit dem Stickstoffatom, an das sie gebunden sind, auch einen 5-, 6- oder 7-gliedrigen, gesättigten N-Heterocyclus bilden können, der ein weiteres Heteroatom, ausgewählt unter O, N und S aufweisen kann und/oder durch 1 bis 4 C₁-C₆-Alkylgruppen substituiert sein kann;
- R⁸: für C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, Hydroxy-C₁-C₄-alkyl, Hydroxycarbonyl-C₁-C₄-alkyl, C₁-C₄-Alkoxy-C₁-C₄-alkyl oder C₁-C₄-Alkoxycarbonyl-C₁-C₄-alkyl steht;
sowie die landwirtschaftlich brauchbaren Salze der Oxazin(thi)on-verbindungen I.

Außerdem betrifft die Erfindung
- die Verwendung von Verbindungen I und ihrer Salze zur Bekämpfung pflanzenpathogener Pilze,
- Mittel, welche die Verbindungen I und/oder ihre Salze in einer fungizid wirksamen Menge, vorzugsweise zusammen mit wenigstens einen Trägerstoff enthalten,
- Verfahren zur Bekämpfung von pflanzenpathogenen Pilzen, bei dem man die Pilze oder die von Pilzbefall bedrohten Materialien, Pflanzen, Saatgüter oder den Erdboden mit einer fungizid wirksamen Menge mindestens einer Verbindung der Formel I gemäß Anspruch 1 und/oder eines Salzes von I behandelt.

Die Verbindungen der Formel I können hinsichtlich der Doppelbindung der Imino-Gruppe sowohl E-Konfiguration, d.h. der Sauerstoff des Oxazinonrings und der Sauerstoff am Imino-Stickstoff stehen in trans-Stellung bezüglich der C=N-Doppelbindung, als auch eine Z-Konfiguration (cis-Anordnung von Sauerstoff des Oxazinonrings und Sauerstoff am Imino-Stickstoff) aufweisen. Gegenstand der Erfindung sind sowohl die E- und die Z-Isomere von I als auch deren Mischungen. Die Substituenten R¹ bis R³ können ein oder mehrere Chiralitätszentren aufweisen. Sie liegen dann als Enantiomeren- oder Diastereomerengemische vor. Gegenstand der Erfindung sind sowohl die reinen Enantiomeren oder Diastereomeren als auch deren Gemische.

Die bei der Definition der Substituenten R¹, R², R³, R⁴, R⁵, R⁶, R⁷ und R⁸ oder als Reste an Cycloalkyl-, Phenyl- oder heterocyclischen Ringen genannten organischen Molekülteile stellen - wie die Bedeutung Halogen - Sammelbegriffe für individuelle Aufzählungen der einzelnen Gruppenmitglieder dar. Sämtliche Kohlenstoffketten, also alle Alkyl-, Halogenalkyl-, Alkoxy-, Halogenalkoxy-, Alkoxycarbonyl, (Halogen)Alkenyl- und (Halogen)Alkinyl-Gruppen sowie entsprechende Gruppenteile in größeren Gruppen wie Phenylalkyl-, Cycloalkylalkyl-, Alkoxyalkyl, Alkoxycarbonylalkyl etc. können geradkettig oder verzweigt sein, wobei das Präfix Cₙ-Cₘ jeweils die mögliche Anzahl von Kohlenstoffatomen in der Gruppe angibt. Halogenierte Substituenten tragen vorzugsweise ein, zwei, drei, vier oder fünf gleiche oder verschiedene Halogenatome.

Im Einzelnen bedeuten beispielsweise:
- Halogen: Fluor, Chlor, Brom, Jod;
- C₁-C₃-Alkyl: Methyl, Ethyl, n-Propyl, 1-Methylethyl (= Isopropyl);
- C₁-C₆-Alkyl: C₁-C₃-Alkyl wie vorstehend genannt, sowie z.B. n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, vorzugsweise Methyl, Ethyl, n-Propyl, 1-Methylethyl, n-Butyl, 1,1-Dimethylethyl, n-Pentyl oder n-Hexyl;
- C₁-C₃-Halogenalkyl: einen Alkylrest mit 1 bis 3 C-Atomen, wie vorstehend genannt, der partiell oder vollständig durch Fluor, Chlor, Brom und/oder Iod und vorzugsweise durch Fluor und/oder Chlor substituiert (Fluor-, Chlor- bzw. Fluor/Chloralkyl) ist, also z.B. CH₂F, CHF₂, CF₃, CH₂Cl, Dichlormethyl, Trichlormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, C₂F₅, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, oder 1-(Brommethyl)-2-bromethyl;
- C₁-C₆-Halogenalkyl: einen Alkylrest mit 1 bis 6 und vorzugsweise 1 bis 4 C-Atomen, wie vorstehend genannt, der partiell oder vollständig durch Fluor, Chlor, Brom und/oder Iod substituiert ist, also z.B. einer der unter C₁-C₃-Halogenalkyl genannten Reste sowie 4-Fluorbutyl, 4-Chlorbutyl, 4-Brombutyl, Nonafluorbutyl, 5-Fluor-1-pentyl, 5-Chlor-1-pentyl, 5-Brom-1-pentyl, 5-Iod-1-pentyl, 5,5,5-Trichlor-1-pentyl, Undecafluorpentyl, 6-Fluor-1-hexyl, 6-Chlor-1-hexyl, 6-Brom-1-hexyl, 6-Iod-1-hexyl, 6,6,6-Trichlor-1-hexyl oder Dodecafluorhexyl. Besonders bevorzugt sind hierunter Trifluormethyl, Trichlormethyl, Dichlorfluormethyl, Difluormethyl, Difluorchlormethyl und 2,2,2-Trifluorethyl;
- C₁-C₄-Alkoxy: OCH₃, OC₂H₅, n-Propoxy, OCH(CH₃)₂, n-Butoxy, OCH(CH₃)-C₂H₅, OCH₂-CH(CH₃)₂, OC(CH₃)₃, vorzugsweise für OCH₃, OC₂H₅, OCH(CH₃)₂;
- C₁-C₆-Alkoxy: C₁-C₄-Alkoxy wie vorstehend definiert sowie für n-Pentyl, 1-Methylbutyloxy, 2-Methylbutyloxy, 3-Methylbutyloxy, 2,2-Dimethylpropyloxy, 1-Ethylpropyloxy, n-Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethylbutyloxy, 2-Ethylbutyloxy, 1,1,2-Trimethylpropyloxy, 1,2,2-Trimethylpropyloxy, 1-Ethyl-1-methylpropyloxy oder 1-Ethyl-2-methylpropyloxy, vorzugsweise Methoxy, Ethoxy, n-Propyloxy, 1-Methylethyl, n-Butoxy, 1,1-Dimethylethyloxy, n-Pentyloxy oder n-Hexyloxy;
- C₁-C₄-Halogenalkoxy: einen C₁-C₄-Alkoxyrest wie vorstehend genannt, der partiell oder vollständig durch Fluor, Chlor, Brom und/oder Iod, vorzugsweise durch Fluor substituiert ist, also z.B. OCH₂F, OCHF₂, OCF₃, OCH₂Cl, OCH(Cl)₂, OC(Cl)₃, Chlorfluormethoxy, Dichlorfluormethoxy, Chlordifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, OC₂F₅, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, 2,2,3,3,3-Pentafluorpropoxy (OCF₂-C₂F₅), 1-(CH₂F)-2-fluorethoxy, 1-(CH₂Cl)-2-chlorethoxy, 1-(CH₂Br)-2-bromethoxy, 4-Fluorbutoxy, 4-Chlorbutoxy, 4-Brombutoxy oder Nonafluorbutoxy, vorzugsweise für OCHF₂, OCF₃, Dichlorfluormethoxy, Chlordifluormethoxy, 2,2,2-Trifluorethoxy;
- C₁-C₆-Halogenalkoxy: einen C₁-C₆-Alkoxyrest wie vorstehend genannt, der partiell oder vollständig durch Fluor, Chlor, Brom und/oder Iod, vorzugsweise durch Fluor substituiert ist, also z.B. für einen der vorstehend genannten C₁-C₄-Halogenalkoxy-Reste sowie für 5-Fluor-1-pentyloxy, 5-Chlor-1-pentyloxy, 5-Brom-1-pentyl, 5-Iod-1-pentyl, 5,5,5-Trichlor-1-pentyloxy, Undecafluorpentyloxy, 6-Fluor-1-hexyloxy, 6-Chlor-1-hexyloxy, 6-Brom-1-hexyloxy, 6-Iod-1-hexyloxy, 6,6,6-Trichlor-1-hexyloxy oder Dodecafluorhexyloxy;
- Phenyl-C₁-C₄-alkyl: C₁-C₄-Alkyl, das einen Phenylring trägt, z.B. für Benzyl, 1-Phenylethyl, 2-Phenylethyl, 1-Phenylprop-1-yl, 2-Phenylprop-1-yl, 3-Phenylprop-1-yl, 1-Phenylbut-1-yl, 2-Phenylbut-1-yl, 3-Phenylbut-1-yl, 4-Phenylbut-1-yl, 1-Phenylbut-2-yl, 2-Phenylbut-2-yl, 3-Phenylbut-2-yl, 4-Phenylbut-2-yl, 1-(Phenylmethyl)-eth-1-yl, 1-(Phenylmethyl)-1-(methyl)-eth-1-yl oder 1-(Phenylmethyl)-prop-1-yl, vorzugsweise Benzyl oder 2-Phenylethyl;
- C₁-C₄-Alkoxy-C₁-C₄-alkyl: durch C₁-C₄-Alkoxy - wie vorstehend genannt - substituiertes C₁-C₄-Alkyl, also z.B. für CH₂-OCH₃, CH₂-OC₂H₅, n-Propoxymethyl, CH₂-OCH(CH₃)₂, n-Butoxymethyl, (1-Methylpropoxy)methyl, (2-Methylpropoxy)methyl, CH₂-OC(CH₃)₃, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)propyl, 3-(n-Propoxy)propyl, 3-(1-Methylethoxy)propyl, 3-(n-Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl, vorzugsweise für CH₂-OCH₃, CH₂-OC₂H₅, 2-Methoxyethyl oder 2-Ethoxyethyl;
- Hydroxy-C₁-C₄-alkyl: C₁-C₄-Alkyl, vorzugsweise C₂-C₄-Alkyl, das eine OH-Gruppe trägt, z.B. für Hydroxymethyl, 2-Hydroxyeth-1-yl, 2-Hydroxy-prop-1-yl, 3-Hydroxy-prop-1-yl, 1-Hydroxy-prop-2-yl, 2-Hydroxy-but-1-yl, 3-Hydroxy-but-1-yl, 4-Hydroxy-but-1-yl, 1-Hydroxy-but-2-yl, 1-Hydroxy-but-3-yl, 2-Hydroxy-but-3-yl, 1-Hydroxy-2-methyl-prop-3-yl, 2-Hydroxy-2-methyl-prop-3-yl oder 2-Hydroxymethyl-prop-2-yl, insbesondere für 2-Hydroxyethyl;
- Hydroxycarbonyl-C₁-C₄-alkyl: C₁-C₄-Alkyl, vorzugsweise C₂-C₄-Alkyl, das eine COOH-Gruppe trägt, z.B. für Hydroxycarbonylmethyl, 2-Hydroxycarbonyleth-1-yl, 2-Hydroxycarbonylprop-1-yl, 3-Hydroxycarbonylprop-1-yl, 1-Hydroxycarbonylprop-2-yl, 2-Hydroxycarbonylbut-1-yl, 3-Hydroxycarbonylbut-1-yl, 4-Hydroxycarbonylbut-1-yl, 1-Hydroxycarbonylbut-2-yl, 1-Hydroxycarbonylbut-3-yl, 2-Hydroxycarbonylbut-3-yl, 1-Hydroxycarbonyl-2-methyl-prop-3-yl, 2-Hydroxycarbonyl-2-methyl-prop-3-yl oder 2-Hydroxycarbonyl-1-methylprop-2-yl, insbesondere für 2-Hydroxycarbonylethyl;
- (C₁-C₄-Alkoxy)carbonyl-C₁-C₄-alkyl: durch (C₁-C₄-Alkoxy)carbonyl wie CO-OCH₃, CO-OC₂H₅, CO-OCH₂-C₂H₅, CO-OCH(CH₃)₂, n-Butoxycarbonyl, CO-OCH(CH₃)-C₂H₅, CO-OCH₂-CH(CH₃)₂ oder CO-OC(CH₃)₃, vorzugsweise CO-OCH₃ oder CO-OC₂H₅ substituiertes C₁-C₄-Alkyl, also z.B. für CH₂-CO-OCH₃, CH₂-CO-OC₂H₅, CH₂-CO-OCH₂-C₂H₅, CH₂-CO-OCH(CH₃)₂, n-Butoxycarbonylmethyl, CH₂-CO-OCH(CH₃)-C₂H₅, CH₂-CO-OCH₂-CH(CH₃)₂, CH₂-CO-OC(CH₃)₃, 1-(CO-OCH₃)ethyl, 1-(CO-OC₂H₅)ethyl, 1-(CO-OCH₂-C₂H₅)ethyl, 1-[CH(CH₃)₂]ethyl, 1-(n-Butoxycarbonyl)ethyl, 1-[1-Methylpropoxycarbonyl)ethyl, 1-[2-Methylpropoxycarbonyl]ethyl, 2-(CO-OCH₃)ethyl, 2-(CO-OC₂H₅)ethyl, 2-(CO-OCH₂-C₂H₅)ethyl, 2-[CO-OCH(CH₃)₂]ethyl, 2-(n-Butoxycarbonyl)ethyl, 2-[1-Methylpropoxycarbonyl]ethyl, 2-[2-Methylpropoxycarbonyl]ethyl, 2-[CO-OC(CH₃)₃]ethyl, 2-(CO-OCH₃)propyl, 2-(CO-OC₂H₅)propyl, 2-(CO-OCH₂-C₂H₅)propyl, 2-[CO-OCH(CH₃)₂]propyl, 2-(n-Butoxycarbonyl)propyl, 2-[1-Methylpropoxycarbonyl]propyl, 2-[2-Methylpropoxycarbonyl]propyl, 2-[CO-OC(CH₃)₃]propyl, 3-(CO-OCH₃)-propyl, 3-(CO-OC₂H₅)propyl, 3-(CO-OCH₂-C₂H₅)propyl, 3-[CO-OCH(CH₃)₂]propyl, 3-(n-Butoxycarbonyl)propyl, 3-[1-Methylpropoxycarbonyl]propyl, 3-[2-Methylpropoxycarbonyl]propyl, 3-[CO-OC(CH₃)₃]propyl, 2-(CO-OCH₃)butyl, 2-(CO-OC₂H₅)butyl, 2-(CO-OCH₂-C₂H₅)butyl, 2-[CO-OCH(CH₃)₂]butyl, 2-(n-Butoxycarbonyl)butyl, 2-[1-Methylpropoxycarbonyl]butyl, 2-[2-Methylpropoxycarbonyl]butyl, 2-[CO-OC(CH₃)₃]butyl, 3-(CO-OCH₃)butyl, 3-(CO-OC₂H₅)butyl, 3-(CO-OCH₂-C₂H₅)butyl, 3-[CO-OCH(CH₃)₂]butyl, 3-(n-Butoxycarbonyl)butyl, 3-[1-Methylpropoxycarbonyl]butyl, 3-[2-Methylpropoxycarbonyl]butyl, 3-[CO-OC(CH₃)₃]butyl, 4-(CO-OCH₃)butyl, 4-(CO-OC₂H₅)butyl, 4-(CO-OCH₂-C₂H₅)butyl, 4-[CO-OCH(CH₃)₂]butyl, 4-(n-Butoxycarbonyl)butyl, 4-[1-Methylpropoxycarbonyl]butyl, 4-[2-Methylpropoxycarbonyl]butyl oder 4-[CO-OC(CH₃)₃]butyl, vorzugsweise für CH₂-CO-OCH₃, CH₂-CO-OC₂H₅, 1-(CO-OCH₃)ethyl oder 1-(CO-OC₂H₅)ethyl;
- C₂-C₆-Alkenyl: geradkettige oder verzweigte Kohlenwasserstoffreste mit 2 bis 6 und vorzugsweise 3 bis 4 Kohlenstoffatomen und einer Doppelbindung in einer beliebigen Position, z.B. Ethenyl (Vinyl), Prop-1-en-1-yl, Allyl, 1-Methylethenyl, 1-Buten-1-yl, 1-Buten-2-yl, 1-Buten-3-yl, 2-Buten-1-yl, 1-Methyl-prop-1-en-1-yl, 2-Methyl-prop-1-en-1-yl, 1-Methyl-prop-2-en-1-yl, 2-Methyl-prop-2-en-1-yl, n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl;
- C₂-C₆-Alkinyl: geradkettige oder verzweigte Kohlenwasserstoffgruppen mit 2 bis 6 und vorzugsweise 3 bis 4 Kohlenstoffatomen und einer Dreifachbindung in einer beliebigen Position, z. B. Ethinyl und C₃-C₆-Alkinyl wie Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Rex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl, vorzugsweise für Prop-2-in-1-yl;
- C₂-C₆-Halogenalkenyl: C₂-C₆-Alkenyl, vorzugsweise C₃-C₄-Alkenyl wie vorstehend genannt, das partiell oder vollständig, vorzugsweise 1-, 2- oder 3-fach durch Halogen, insbesondere durch Fluor, Chlor und/oder Brom substituiert ist, also z.B. 2-Chlorvinyl, 2-Chlorallyl, E- und Z-3-Chlorallyl, E- und Z-2,3-Dichlorallyl, 3,3-Dichlorallyl, 2,3,3-Trichlorallyl, E- und Z-2,3-Dichlorbut-2-enyl, 2-Bromallyl, E- und Z-3-Bromallyl, E- und Z-2,3-Dibromallyl, 3,3-Dibromallyl, 2,3,3-Tribromallyl sowie E- und Z-2,3-Dibrombut-2-enyl;
- C₂-C₆-Halogenalkinyl: C₂-C₆-Alkinyl, vorzugsweise C₃-C₄-Alkinyl wie vorstehend genannt, das partiell oder vollständig, vorzugsweise 1-, 2- oder 3-fach, durch Halogen, insbesondere durch Fluor, Chlor und/oder Brom substituiert ist, also z.B. 1,1-Difluorprop-2-in-1-yl, 1,1-Difluorbut-2-in-1-yl, 4-Fluorbut-2-in-1-yl, 4-Chlorbut-2-in-1-yl, 5-Fluorpent-3-in-1-yl oder 6-Fluorhex-4-in-1-yl;
- C₃-C₈-Cycloalkyl: Cyclopropyl, Cyclobutyl, Cyclopentyl, Cyclohexyl, Cycloheptyl oder Cyclooctyl;
- C₃-C₈-Cycloalkyl-C₁-C₄-alkyl: C₁-C₄-Alkyl, das einen C₃-C₈-Cycloalkyl-Rest trägt, z.B. für Cyclopropylmethyl, 1-Cyclopropyl-ethyl, 2-Cyclopropyl-ethyl, 1-Cyclopropyl-prop-1-yl, 2-Cyclopropyl-prop-1-yl, 3-Cyclopropyl-prop-1-yl, 1-Cyclopropyl-but-1-yl, 2-Cyclopropyl-but-1-yl, 3-Cyclopropyl-but-1-yl, 4-Cyclopropyl-but-1-yl, 1-Cyclopropyl-but-2-yl, 2-Cyclopropyl-but-2-yl, 3-Cyclopropyl-but-2-yl, 3-Cyclopropyl-but-2-yl, 4-Cyclopropyl-but-2-yl, 1-(Cyclopropylmethyl)-eth-1-yl, 1-(Cyclopropylmethyl)-1-(methyl)-eth-1-yl, 1-(Cyclopropylmethyl)-prop-1-yl, Cyclobutylmethyl, 1-Cyclobutyl-ethyl, 2-Cyclobutyl-ethyl, 1-Cyclobutyl-prop-1-yl, 2-Cyclobutyl-prop-1-yl, 3-Cyclobutyl-prop-1-yl, 1-Cyclobutyl-but-1-yl, 2-Cyclobutyl-but-1-yl, 3-Cyclobutyl-but-1-yl, 4-Cyclobutyl-but-1-yl, 1-Cyclobutyl-but-2-yl, 2-Cyclobutyl-but-2-yl, 3-Cyclobutyl-but-2-yl, 3-Cyclobutyl-but-2-yl, 4-Cyclobutyl-but-2-yl, 1-(Cyclobutylmethyl)-eth-1-yl, 1-(Cyclobutylmethyl)-1-(methyl)-eth-1-yl, 1-(Cyclobutylmethyl)-prop-1-yl, Cyclopentylmethyl, 1-Cyclopentyl-ethyl, 2-Cyclopentyl-ethyl, 1-Cyclopentyl-prop-1-yl, 2-Cyclopentyl-prop-1-yl, 3-Cyclopentyl-prop-1-yl, 1-Cyclopentyl-but-1-yl, 2-Cyclopentyl-but-1-yl, 3-Cyclopentyl-but-1-yl, 4-Cyclopentyl-but-1-yl, 1-Cyclopentyl-but-2-yl, 2-Cyclopentyl-but-2-yl, 3-Cyclopentyl-but-2-yl, 3-Cyclopentyl-but-2-yl, 4-Cyclopentyl-but-2-yl, 1-(Cyclopentylmethyl)-eth-1-yl, 1-(Cyclopentylmethyl)-1-(methyl)-eth-1-yl, 1-(Cyclopentylmethyl)-prop-1-yl, Cyclohexylmethyl, 1-Cyclohexyl-ethyl, 2-Cyclohexyl-ethyl, 1-Cyclohexyl-prop-1-yl, 2-Cyclohexyl-prop-1-yl, 3-Cyclohexyl-prop-1-yl, 1-Cyclohexyl-but-1-yl, 2-Cyclohexyl-but-1-yl, 3-Cyclohexyl-but-1-yl, 4-Cyclohexyl-but-1-yl, 1-Cyclohexyl-but-2-yl, 2-Cyclohexyl-but-2-yl, 3-Cyclohexyl-but-2-yl, 3-Cyclohexyl-but-2-yl, 4-Cyclohexyl-but-2-yl, 1-(Cyclohexylmethyl)-eth-1-yl, 1-(Cyclohexylmethyl)-1-(methyl)-eth-1-yl, 1-(Cyclohexylmethyl)-prop-1-yl, Cycloheptylmethyl, 1-Cycloheptyl-ethyl, 2-Cycloheptyl-ethyl, 1-Cycloheptyl-prop-1-yl, 2-Cycloheptyl-prop-1-yl, 3-Cycloheptyl-prop-1-yl, 1-Cycloheptyl-but-1-yl, 2-Cycloheptyl-but-1-yl, 3-Cycloheptyl-but-1-yl, 4-Cycloheptyl-but-1-yl, 1-Cycloheptyl-but-2-yl, 2-Cycloheptyl-but-2-yl, 3-Cycloheptyl-but-2-yl, 3-Cycloheptyl-but-2-yl, 4-Cycloheptyl-but-2-yl, 1-(Cycloheptylmethyl)-eth-1-yl, 1-(Cycloheptylmethyl)-1-(methyl)-eth-1-yl, 1-(Cycloheptylmethyl)-prop-1-yl, Cyclooctylmethyl, 1-Cyclooctyl-ethyl, 2-Cyclooctyl-ethyl, 1-Cyclooctyl-prop-1-yl, 2-Cyclooctyl-prop-1-yl, 3-Cyclooctyl-prop-1-yl, 1-Cyclooctyl-but-1-yl, 2-Cyclooctyl-but-1-yl, 3-Cyclooctyl-but-1-yl, 4-Cyclooctyl-but-1-yl, 1-Cyclooctyl-but-2-yl, 2-Cyclooctyl-but-2-yl, 3-Cyclooctyl-but-2-yl, 3-Cyclooctyl-but-2-yl, 4-Cyclooctyl-but-2-yl, 1-(Cyclooctylmethyl)-eth-1-yl, 1-(Cyclooctylmethyl)-1-(methyl)-eth-1-yl oder 1-(Cyclooctylmethyl)-prop-1-yl, vorzugsweise für Cyclopropylmethyl, Cyclobutylmethyl, Cyclopentylmethyl oder Cyclohexylmethyl.

Beispiele für 5- oder 6-gliedriges Heterocyclyl mit 1, 2 oder 3 Heteroatomen ausgewählt unter N, O und S, umfassen:
- Ungesättigtes, insbesondere aromatisches Heterocyclyl, z.B. Furyl wie 2-Furyl und 3-Furyl, Thienyl wie 2-Thienyl und 3-Thienyl, Pyrrolyl wie 2-Pyrrolyl und 3-Pyrrolyl, Isoxazolyl wie 3-Isoxazolyl, 4-Isoxazolyl und 5-Isoxazolyl, Isothiazolyl wie 3-Isothiazolyl, 4-Isothiazolyl und 5-Isothiazolyl, Pyrazolyl wie 3-Pyrazolyl, 4-Pyrazolyl und 5-Pyrazolyl, Oxazolyl wie 2-Oxazolyl, 4-Oxazolyl und 5-Oxazolyl, Thiazolyl wie 2-Thiazolyl, 4-Thiazolyl und 5-Thiazolyl, Imidazolyl wie 2-Imidazolyl und 4-Imidazolyl, Oxadiazolyl wie 1,2,4-Oxadiazol-3-yl, 1,2,4-Oxadiazol-5-yl und 1,3,4-Oxadiazol-2-yl, Thiadiazolyl wie 1,2,4-Thiadiazol-3-yl, 1,2,4-Thiadiazol-5-yl und 1,3,4-Thiadiazol-2-yl, Triazolyl wie 1,2,4-Triazol-1-yl, 1,2,4-Triazol-3-yl und 1,2,4-Triazol-4-yl, Pyridinyl wie 2-Pyridinyl, 3-Pyridinyl und 4-Pyridinyl, Pyridazinyl wie 3-Pyridazinyl und 4-Pyridazinyl, pyrimidinyl wie 2-Pyrimidinyl, 4-Pyrimidinyl und 5-pyrimidinyl, des weiteren 2-Pyrazinyl, 1,3,5-Triazin-2-yl und 1,2,4-Triazin-3-yl, insbesondere Pyridyl, Pyrimidyl, Furyl und Thienyl;
- Gesättigtes Heterocyclyl, z.B. Tetrahydrofuran-2-yl, Tetrahydrofuran-3-yl, Tetrahydrothiophen-2-yl, Tetrahydrothiophen-3-yl, Pyrrolidin-1-yl, Pyrrolidin-2-yl, Pyrrolidin-3-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Oxazolidin-2-yl, 1,3-Oxazolidin-3-yl, 1,3-Oxazolidin-4-yl, 1,3-Oxazolidin-5-yl, 1,2-Oxazolidin-2-yl, 1,2-Oxazolidin-3-yl, 1,2-Oxazolidin-4-yl, 1,2-Oxazolidin-5-yl, 1,3-Dithiolan-2-yl, 1,3-Dithiolan-4-yl, Pyrrolidin-1-yl, Pyrrolidin-2-yl, Pyrrolidin-5-yl, Tetrahydropyrazol-1-yl, Tetrahydropyrazol-3-yl, Tetrahydropyrazol-4-yl, Tetrahydropyran-2-yl, Tetrahydropyran-3-yl, Tetrahydropyran-4-yl, Tetrahydrothiopyran-2-yl, Tetrahydrothiopyran-3-yl, Tetrahydropyran-4-yl, Piperidin-1-yl, Piperidin-2-yl, Piperidin-3-yl, Piperidin-4-yl, 1,3-Dioxan-2-yl, 1,3-Dioxan-4-yl, 1,3-Dioxan-5-yl, 1,4-Dioxan-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, Morpholin-2-yl, Morpholin-3-yl, Morpholin-4-yl, Hexahydropyridazin-1-yl, Hexahydropyridazin-3-yl, Hexahydropyridazin-4-yl, Hexahydropyrimidin-1-yl, Hexahydropyrimidin-2-yl, Hexahydropyrimidin-4-yl, Hexahydropyrimidin-5-yl, Piperazin-1-yl, Piperazin-2-yl, Piperazin-3-yl, Hexahydro-1,3,5-triazin-1-yl, Hexahydro-1,3,5-triazin-2-yl.

Substituenten an Phenyl bzw. an Heterocyclyl sind üblicherweise ausgewählt unter Halogen, C₁-C₄-Alkyl, C₁-C₄-Halogenalkyl, C₁-C₄-Alkoxy, C₁-C₄-Halogenalkoxy, Methylendioxy, CN und C₁-C₄-Alkylcarbonyl.

In Formel I bezeichnet die Variable A, bzw. das Strukturelement 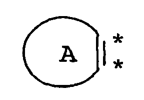 einen über zwei C-Atome mit dem Oxazin(thi)onring anellierten 5- oder 6-gliedrigen Carbocyclus oder einen 5- oder 6-gliedriger Heterocyclus mit 1, 2 oder 3 Heteroatomen, ausgewählt unter N, O und S. In dieser Formeldarstellung der Variable A kennzeichnen die beiden *-Positionen diejenigen C-Atome, die dem Oxazin(thi)on-Ring und dem ihm anellierten Ring gemeinsam sind. Beipiele für anellierte 5- oder 6-gliedrige Carbocyclen sind neben Benzol, auch Cyclohexen, 1,3- und 1,4-Cyclohexandien und Cyclopenten. Beispiele für anellierte 5- oder 6-gliedrige Heterocyclen sind die vorgenannten heteroaromatischen Gruppen, die zwei benachbarte Kohlenstoffringglieder aufweisen, sowie deren Di- oder Tetrahydroderivate, die noch wenigstens eine C=C-Doppelbindung aufweisen, z.B. Pyridin, Pyrazin, Pyridazin, Pyrimidin, Furan, Dihydrofuran, Thiophen, Thiophendioxid, 2,3- und 2,5-Dihydrothiophen, 2,3- und 2,5-Dihydrothiophendioxid, Pyrrol, Dihydropyrrol, 1,3-Dioxolan, Isoxazol, Oxazol, 2,3-Dihydrooxazol, Isothiazol, Thiazol, Pyrazol, Pyrazolin, Imidazol, 2,3-Dihydromidazol, 1,2,3-Triazol, 1,1-Dioxo-2,3-dihydroisothiazol, 2,3-Dihydro-1,4-dioxin, 2,3-Dihydro-1,4-oxazin, 2,4- bzw. 2,6-Dihydro-1,3-oxazin, 2,3-Dihydro-1,4-thiazin und 2,4-Dihydro-1,3-thiazin.

Bevorzugte anellierte Ringe A sind aromatisch und sind insbesondere ausgewählt unter den nachstehend angegebenen Ringen der Formeln A1 bis A37:

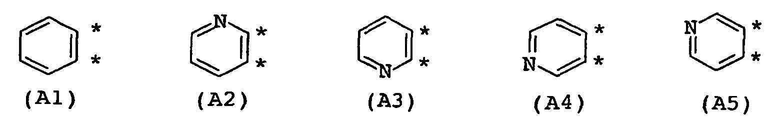

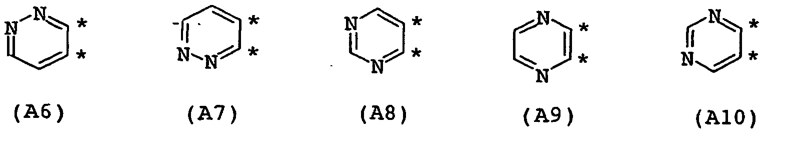

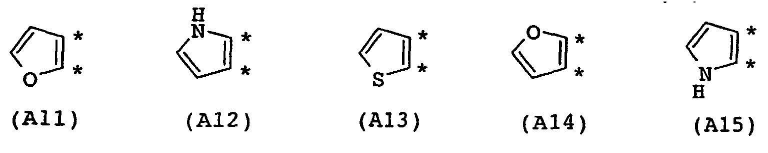

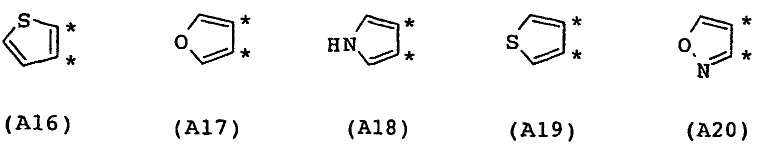

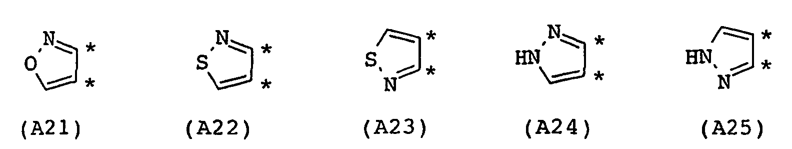

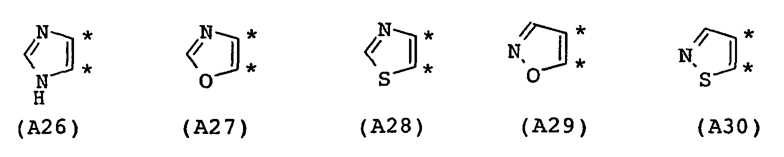

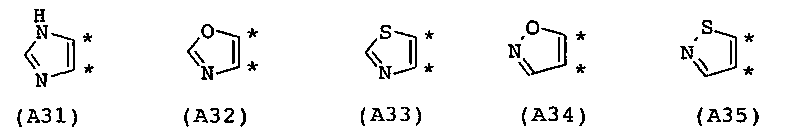

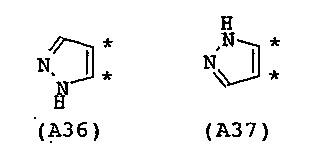

Hierbei sind jeweils die Ringe A24 und A37, A25 und A36 sowie A26 und A31 zueinander tautomer.

In den erfindungsgemäßen Verbindungen der allgemeinen Formel I können die Reste A, z.B. die Reste A1 bis A37, unsubstituiert sein oder, je nach Anzahl der freien Valenzen 1, 2, 3 oder 4, vorzugsweise 1 oder 2 gleiche oder voneinander verschiedene Substituenten R³ tragen. Die Anzahl der Substituenten R³ wird dabei durch die Variable n angegeben. In den Resten der Formeln A12, A15, A18, A24, A25, A26, A31, A36 und A37 kann auch das am Stickstoff befindliche Wasserstoffatom durch einen Substituenten R³ ersetzt sein, wobei in diesem Fall R³ für Alkyl oder Halogenalkyl steht.

Bevorzugt tragen 6-gliedrige Ringe A wie Benzol oder Pyridin in der 6-Position (meta-Position zu dem Kohlenstoffatom, welches die Gruppe C=Z trägt) einen Substituenten R³, sofern diese Position kein Heteroatom aufweist.

Im Hinblick auf die fungizide Wirksamkeit der Verbindungen I weisen die Substituenten Z, R¹, R², R³ und der Index n unabhängig voneinander, vorzugsweise jedoch in Kombination miteinander die folgenden Bedeutungen auf:
- Z: Sauerstoff;
- R¹: C₁-C₆-Alkyl, C₂-C₆-Alkenyl, C₁-C₆-Halogenalkyl, C₂-C₆-Halogenalkenyl, C₁-C₆-Alkyl, das durch OR⁴ oder C₃-C₈-Cycloalkyl-C₁-C₄-alkyl substituiert ist. Hierin hat R⁴ die zuvor genannten Bedeutungen und steht insbesondere für C₁-C₄-Alkyl. R¹ steht insbesondere für C₃-C₆-Alkyl, C₃-C₄-Alkenyl, C₃-C₄-Alkinyl oder C₃-C₆-Cylcoalkylmethyl;
- R²: C₁-C₃-Alkyl, C₁-C₃-Fluoralkyl, C₁-C₃-Fluor/Chloralkyl oder Cyclopropylmethyl;
- R³: C₁-C₄-Alkyl, insbesondere Methyl, C₁-C₄-Halogenalkyl, insbesondere Difluormethyl, Trifluormethyl oder 2,2,2-Trifluorethyl, C₁-C₄-Halogenalkoxy, insbesondere Difluormethoxy, oder besonders bevorzugt Halogen. Ganz besonders bevorzugt ist Chlor, Brom oder Iod;
- n: 1, 2, 3 oder 4, insbesondere 1 oder 2.

Die anellierte Cyclus A ist insbesondere ausgewählt unter Benzol (Formel A1), Pyridin (Formeln A2 bis A5) und Thiophen (Formeln A13, A16 und A19).

Sofern R¹ für ein durch COOR⁵ substituiertes Alkyl, Alkenyl oder Alkinyl steht, bedeutet R⁵ vorzugsweise C₁-C₄-Alkyl. Sofern R¹ für ein durch C(O)NR⁶R⁷ substituiertes C₁-C₆-Alkyl, -Alkenyl oder -Alkinyl steht, bedeutet R⁶ vorzugsweise Wasserstoff oder C₁-C₄-Alkyl und R⁷ vorzugsweise Wasserstoff. Sofern R¹ für ein durch NR⁶R⁷ substituiertes C₁-C₆-Alkyl, -Alkenyl oder -Alkinyl steht, bedeuten R⁶ und R⁷ unabhängig voneinander vorzugsweise Wasserstoff oder C₁-C₄-Alkyl oder R⁶ und R⁷ bilden gemeinsam mit dem Stickstoffatom, an das sie gebunden sind einen Morpholin-, Piperidin-, Piperazin- oder Pyrrolidin-Ring. Sofern R¹ für ein durch SR⁸ substituiertes C₁-C₆-Alkyl, -Alkenyl oder -Alkinyl steht, bedeutet R⁸ vorzugsweise C₁-C₄-Alkyl.

Besonders bevorzugte Verbindungen I sind die Verbindungen der allgemeinen Formel I-A1 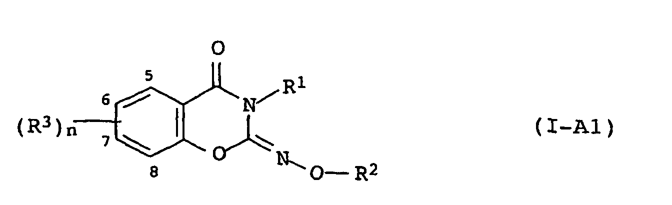 worin n, R¹, R² und R³ die folgenden Bedeutungen aufweisen:
- R¹: C₁-C₆-Alkyl, C₂-C₆-Alkenyl, C₁-C₆-Halogenalkyl, C₂-C₆-Halogenalkenyl, C₁-C₄-Alkoxy-C₁-C₄-alkyl oder C₃-C₆-Cycloalkyl-C₁-C₄-alkyl, speziell C₃-C₆-Alkyl, C₃-C₄-Alkenyl, C₃-C₄-Halogenalkenyl, C₁-C₄-Alkoxyalkyl und C₃-C₆-Cycloalkylmethyl;
- R²: C₁-C₃-Alkyl oder C₁-C₃-Halogenalkyl;
- R³: C₁-C₄-Alkyl, C₁-C₄-Halogenalkyl, insbesondere Difluormethyl, Trifluormethyl oder 2,2,2-Trifluorethyl, C₁-C₄-Halogenalkoxy, insbesondere Difluormethoxy, oder besonders bevorzugt Halogen. Ganz besonders bevorzugt ist Chlor, Brom oder Iod;
- n: für 1 oder 2.

Hierunter besonders bevorzugt sind Verbindungen I-A1, in denen einer der Substituenten R³ in der 6-Position steht.

Besonders bevorzugt sind auch Verbindungen der Formel I-A1, worin R² für Cyclopropylmethyl steht und R¹, R³ und n die zuvor angegebenen Bedeutungen aufweisen.

Beispiele für besonders bevorzugte Verbindungen der Formel I-A1 sind die Verbindungen I-A1.1 bis I-A1.128, worin die Variablen R¹, R² und R³ jeweils die in einer Zeile der Tabelle 1 angegebenen Bedeutungen haben.

**Tabelle 1:**

| | R¹ | R² | n | R³ |
|---|---|---|---|---|
| I-A1.1 | n-Propyl | Methyl | 1 | 6-Fluor |
| I-A1.2 | n-Propyl | Methyl | 1 | 6-Chlor |
| I-A1.3 | n-Propyl | Methyl | 1 | 6-Brom |
| I-A1.4 | n-Propyl | Methyl | 1 | 6-Iod |
| I-A1.5 | n-Propyl | Ethyl | 1 | 6-Fluor |
| I-A1.6 | n-Propyl | Ethyl | 1 | 6-Chlor |
| I-A1.7 | n-Propyl | Ethyl | 1 | 6-Brom |
| I-A1.8 | n-Propyl | Ethyl | 1 | 6-Iod |
| I-A1.9 | n-Propyl | n-Propyl | 1 | 6-Fluor |
| T-A1.10 | n-Propyl | n-Propyl | 1 | 6-Chlor |
| I-A1.11 | n-Propyl | n-Propyl | 1 | 6-Brom |
| I-A1.12 | n-Propyl | n-Propyl | 1 | 6-Iod |
| I-A1.13 | n-Propyl | 2,2,2-Trifluormethyl | 1 | 6-Fluor |
| I-A1.14 | n-Propyl | 2,2,2-Trifluormethyl | 1 | 6-Chlor |
| I-A1.15 | n-Propyl | 2,2,2-Trifluormethyl | 1 | 6-Brom |
| I-A1.16 | n-Propyl | 2,2,2-Trifluormethyl | 1 | 6-Iod |
| I-A1.17 | n-Butyl | Methyl | 1 | 6-Fluor |
| I-A1.18 | n-Butyl | Methyl | 1 | 6-Chlor |
| I-A1.19 | n-Butyl | Methyl | 1 | 6-Brom |
| I-A1.20 | n-Butyl | Methyl | 1 | 6-Iod |
| I-A1.21 CCC | n-Butyl | Ethyl | 1 | 6-Fluor |
| I-A1.22 | n-Butyl | Ethyl | 1 | 6-Chlor- |
| I-A1.23 | n-Butyl | Ethyl | 1 | 6-Brom |
| I-A1.24 | n-Butyl | Ethyl | 1 | 6-Iod |
| I-A1.25 | n-Butyl | n-Propyl | 1 | 6-Fluor |
| I-A1.26 | n-Butyl | n-Propyl | 1 | 6-Chlor |
| I-A1.27 | n-Butyl | n-Propyl | 1 | 6-Brom |
| I-A1.28 | n-Butyl | n-Propyl | 1 | 6-Iod |
| I-A1.29 | n-Butyl | 2,2,2-Trifluormethyl | 1 | 6-Fluor |
| I-A1.30 | n-Butyl | 2,2,2-Trifluormethyl | 1 | 6-Chlor |
| I-A1.31 | n-Butyl | 2,2,2-Trifluormethyl | 1 | 6-Brom |
| I-A1.32 | n-Butyl | 2,2,2-Trifluormethyl | 1 | 6-Iod |
| I-A1.33 | 2-Butyl | Methyl | 1 | 6-Fluor |
| I-A1.34 | 2-Butyl | Methyl | 1 | 6-Chlor |
| I-A1.35 | 2-Butyl | Methyl | 1 | 6-Brom |
| I-A1.36 | 2-Butyl | Methyl | 1 | 6-Iod |
| I-A1.37 | 2-Butyl | Ethyl | 1 | 6-Fluor |
| I-A1.38 | 2-Butyl | Ethyl | 1 | 6-Chlor |
| I-A1.39 | 2-Butyl | Ethyl | 1 | 6-Brom |
| I-A1.40 | 2-Butyl | Ethyl | 1 | 6-Iod |
| I-A1.41 | 2-Butyl | n-Propyl | 1 | 6-Fluor |
| I-AI.42 | 2-Butyl | n-Propyl | 1 | 6-Chlor |
| I-A1.43 | 2-Butyl | n-Propyl | 1 | 6-Brom |
| I-A1.44 | 2-Butyl | n-Propyl | 1 | 6-Iod |
| I-A1.45 | 2-Butyl | 2,2,2-Trifluormethyl | 1 | 6-Fluor |
| I-A1.46 | 2-Butyl | 2,2,2-Trifluormethyl | 1 | 6-Chlor |
| I-A1.47 | 2-Butyl | 2,2,2-Trifluormethyl | 1 | 6-Brom |
| I-A1.48 | 2-Butyl | 2,2,2-Trifluormethyl | 1 | 6-Iod |
| I-A1.49 | Isobutyl | Methyl | 1 | 6-Fluor |
| I-A1.50 | Isobutyl | Methyl | 1 | 6-Chlor |
| I-A1.51 | Isobutyl | Methyl | 1 | 6-Brom |
| I-A1.52 | Isobutyl | Methyl | 1 | 6-Iod |
| I-A1.53 | Isobutyl | Ethyl | 1 | 6-Fluor |
| I-A1.54 | Isobutyl | Ethyl | 1 | 6-Chlor |
| I-A1.55 | Isobutyl | Ethyl | 1 | 6-Brom |
| I-A1.56 | Isobutyl | Ethyl | 1 | 6-Iod |
| I-A1.57 | Isobutyl | n-Propyl | 1 | 6-Fluor |
| I-A1.58 | Isobutyl | n-Propyl | 1 | 6-Chlor |
| I-A1.59 | Isobutyl | n-Propyl | 1 | 6-Brom |
| I-A1.60 | Isobutyl | n-Propyl | 1 | 6-Iod |
| I-A1.61 | Isobutyl | 2,2,2-Trifluormethyl | 1 | 6-Fluor |
| I-A1.62 | Isobutyl | 2,2,2-Trifluormethyl | 1 | 6-Chlor |
| I-A1.63 | Isobutyl | 2,2,2-Trifluormethyl | 1 | 6-Brom |
| I-A1.64 | Isobutyl | 2,2,2-Trifluormethyl | 1 | 6-Iod |
| I-A1.65 | Cyclopropylmethyl | Methyl | 1 | 6-Fluor |
| 1-A1.66 | Cyclopropylmethyl | Methyl | 1 | 6-Chlor |
| I-A1.67 | Cyclopropylmethyl | Methyl | 1 | 6-Brom |
| I-A1.68 | Cyclopropylmethyl | Methyl | 1 | 6-Iod |
| I-A1.69 | Cyclopropylmethyl | Ethyl | 1 | 6-Fluor |
| I-A1.70 | Cyclopropylmethyl | Ethyl | 1 | 6-Chlor |
| I-A1.71 | Cyclopropylmethyl | Ethyl | 1 | 6-Brom |
| I-A1.72 | Cyclopropylmethyl | Ethyl | 1 | 6-Iod |
| I-A1.73 | Cyclopropylmethyl | n-Propyl | 1 | 6-Fluor |
| I-A1.74 | Cyclopropylmethyl | n-Propyl | 1 | 6-Chlor |
| I-A1.75 | Cyclopropylmethyl | n-Propyl | 1 | 6-Brom |
| I-A1.76 | Cyclopropylmethyl | n-Propyl | 1 | 6-Iod |
| I-A1.77 | Cyclopropylmethyl | 2,2,2-Trifluormethyl | 1 | 6-Fluor |
| I-A1.78 | Cyclopropylmethyl | 2,2,2-Trifluormethyl | 1 | 6-Chlor |
| I-A1.79 | Cyclopropylmethyl | 2,2,2-Trifluormethyl | 1 | 6-Brom |
| I-A1.80 | Cyclopropylmethyl | 2,2,2-Trifluormethyl | 1 | 6-Iod |
| I-A1.81 | E-3-Chlor-2-propenyl | Methyl | 1 | 6-Fluor |
| I-A1.82 | E-3-Chlor-2-propenyl | Methyl | 1 | 6-Chlor |
| I-A1.83 | E-3-Chlor-2-propenyl | Methyl | 1 | 6-Brom |
| I-A1.84 | E-3-Chlor-2-propenyl | Methyl | 1 | 6-Iod |
| I-A1.85 | E-3-Chlor-2-propenyl | Ethyl | 1 | 6-Fluor |
| I-A1.86 | E-3-Chlor-2-propenyl | Ethyl | 1 | 6-Chlor |
| I-A1.87 | E-3-Chlor-2-propenyl | Ethyl | 1 | 6-Brom |
| I-A1.88 | E-3-Chlor-2-propenyl | Ethyl | 1 | 6-Iod |
| I-A1.89 | E-3-Chlor-2-propenyl | n-Propyl | 1 | 6-Fluor |
| I-A1.90 | E-3-Chlor-2-propenyl | n-Propyl | 1 | 6-Chlor |
| I-A1.91 | E-3-Chlor-2-propenyl | n-Propyl | 1 | 6-Brom |
| I-A1.92 | E-3-Chlor-2-propenyl | n-Propyl | 1 | 6-Iod |
| I-A1.93 | E-3-Chlor-2-propenyl | 2,2,2-Trifluormethyl | 1 | 6-Fluor |
| I-A1.94 | E-3-Chlor-2-propenyl | 2,2,2-Trifluormethyl | 1 | 6-Chlor |
| I-A1.95 | E-3-Chlor-2-propenyl | 2,2,2-Trifluormethyl | 1 | 6-Brom |
| I-A1.96 | E-3-Chlor-2-propenyl | 2,2,2-Trifluormethyl | 1 | 6-Iod |
| I-A1.97 | CH₂-CH=CH₃ | Methyl | 1 | 6-Fluor |
| I-A1.98 | CH₂-CH=CH₃ | Methyl | 1 | 6-Chlor |
| I-A1.99 | CH₂-CH=CH₃ | Methyl | 1 | 6-Brom |
| I-A1.100 | CH₂-CH=CH₃ | Methyl | 1 | 6-Iod |
| I-A1.101 | CH₂-CH=CH₃ | Ethyl | 1 | 6-Fluor |
| I-A1.102 | CH₂-CH=CH₃ | Ethyl | 1 | 6-Chlor |
| I-A1.103 | CH₂-CH=CH₃ | Ethyl | 1 | 6-Brom |
| I-A1.104 | CH₂-CH=CH₃ | Ethyl | 1 | 6-Iod |
| I-A1.105 | CH₂-CH=CH₃ | n-Propyl | 1 | 6-Fluor |
| I-A1.106 | CH₂-CH=CH₃ | n-Propyl | 1 | 6-Chlor |
| I-A1.107 | CH₂-CH=CH₃ | n-Propyl | 1 | 6-Brom |
| I-A1.108 | CH₂-CH=CH₃ | n-Propyl | 1 | 6-Iod |
| I-A1.109 | CH₂-CH=CH₃ | 2,2,2-Trifluormethyl | 1 | 6-Fluor |
| I-A1.110 | CH₂-CH=CH₃ | 2,2,2-Trifluormethyl | 1 | 6-Chlor |
| I-A1.111 | CH₂-CH=CH₃ | 2,2,2-Trifluormethyl | 1 | 6-Brom |
| I-A1.112 | CH₂-CH=CH₃ | 2,2,2-Trifluormethyl | 1 | 6-Iod |
| I-A1.113 | CH₂C≡CH | Methyl | 1 | 6-Fluor |
| I-A1.114 | CH₂C≡CH | Methyl | 1 | 6-Chlor |
| I-A1.115 | CH₂C≡CH | Methyl | 1 | 6-Brom |
| I-A1.116 | CH₂C≡CH | Methyl | 1 | 6-Iod |
| I-A1.117 | CH₂C≡CH | Ethyl | 1 | 6-Fluor |
| I-A1.118 | CH₂C≡CH | Ethyl | 1 | 6-Chlor |
| I-A1.119 | CH₂C≡CH | Ethyl | 1 | 6-Brom |
| I-A1.120 | CH₂C≡CH | Ethyl | 1 | 6-Iod |
| I-A1.121 | CH₂C≡CH | n-Propyl | 1 | 6-Fluor |
| I-A1.122 | CH₂C≡CH | n-Propyl | 1 | 6-Chlor |
| I-A1.123 | CH₂C≡CH | n-Propyl | 1 | 6-Brom |
| I-A1.124 | CH₂C≡CH | n-Propyl | 1 | 6-Iod |
| I-A1.125 | CH₂C≡CH | 2,2,2-Trifluormethyl | 1 | 6-Fluor |
| I-A1.126 | CH₂C≡CH | 2,2,2-Trifluormethyl | 1 | 6-Chlor |
| I-A1.127 | CH₂C≡CH | 2,2,2-Trifluormethyl | 1 | 6-Brom |
| I-A1.128 | CH₂C≡CH | 2,2,2-Trifluormethyl | 1 | 6-Iod |
| I-A1.129 | n-Propyl | n-Propyl | 0 | |
| I-A1.130 | n-Propyl | n-Propyl | 1 | 6-Methyl |
| I-A1.131 | Methyl | n-Propyl | 1 | 6-Methyl |
| I-A1.132 | n-Propyl | Methyl | 1 | 6-Methyl |
| I-A1.133 | n-Propyl | Methyl | 1 | 6-Methoxy |
| I-A1.134 | Cyclopropylmethyl | 2-Propyl | 1 | 6-Iod |
| I-A1.135 | Cyclopropylmethyl | Cyclopropylmethyl | 1 | 6-Iod |
| I-A1.136 | n-Butyl | Cyclopropylmethyl | 1 | 6-Iod |
| I-A1.137 | n-Propyl | Cyclopropylmethyl | 1 | 6-Iod |
| I-A1.138 | n-Butyl | 2-Propyl | 1 | 6-Iod |
| I-A1.139 | n-Propyl | 2-Propyl | 1 | 6-Iod |

In der Tabelle 1 sowie in den folgenden Tabellen 2 und 3 gibt die dem Substituenten vorangestellte Zahl dessen Position im anellierten Ring A an.

Besonders bevorzugt sind außerdem die Verbindungen der allgemeinen Formel I-A13, I-A16 und I-A19 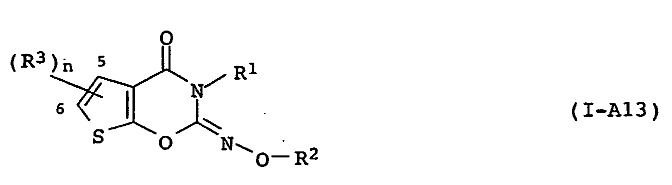 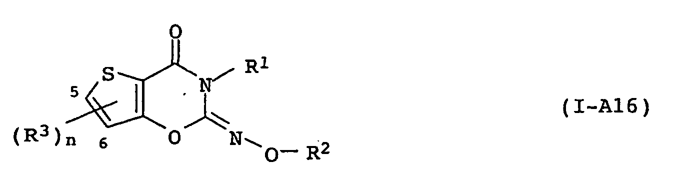 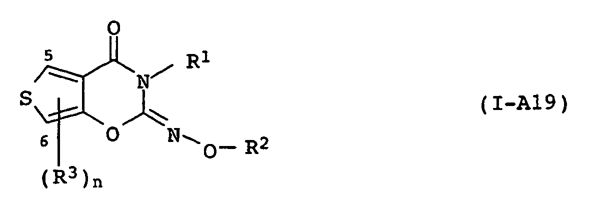 worin n, R¹, R² und R³ die folgenden Bedeutungen aufweisen:
- R¹: C₁-C₆-Alkyl, C₂-C₆-Alkenyl, C₁-C₆-Halogenalkyl, C₂-C₆-Halogenalkenyl, C₁-C₄-Alkoxy-C₁-C₄-alkyl oder C₃-C₆-Cycloalkyl-C₁-C₄-alkyl, speziell C₃-C₆-Alkyl, C₃-C₄-Alkenyl, C₃-C₄-Halogenalkenyl, C₁-C₄-Alkoxyalkyl und C₃-C₆-Cycloalkylmethyl;
- R²: C₁-C₃-Alkyl oder C₁-C₃-Halogenalkyl;
- R³: C₁-C₄-Alkyl, insbesondere Methyl, C₁-C₄-Halogenalkyl, insbesondere Difluormethyl, Trifluormethyl oder 2,2,2-Trifluorethyl, C₁-C₄-Halogenalkoxy, insbesondere Difluormethoxy, oder besonders bevorzugt Halogen. Ganz besonders bevorzugt ist Chlor, Brom oder Iod;
- n: für 1 oder 2.

Besonders bevorzugt sind auch Verbindungen der Formel I-A13, I-A16 und I-A19, worin R² für Cyclopropylmethyl steht und R¹, R³ und n die zuvor angegebenen Bedeutungen aufweisen.

Beispiele für besonders bevorzugte Verbindungen der allgemeinen Formeln I-A13, I-A16 und I-A19 sind die Verbindungen, worin die Variablen R¹, R² und R³ jeweils die in einer Zeile der Tabelle 2 angegebenen Bedeutungen haben (Verbindungen I-A13.1 bis I-A13.18, I-A16.1 bis I-A16.18 und I-A19.1 bis I-A19.18).

**Tabelle 2:**

| | R¹ | R² | n | R³ |
|---|---|---|---|---|
| 1 | n-Propyl | Methyl | 1 | 5-Chlor |
| 2 | n-Propyl | Methyl | 1 | 6-Chlor |
| 3 | n-Propyl | Methyl | 0 | -- |
| 4 | n-Butyl | Methyl | 1 | 5-Chlor |
| 5 | n-Butyl | Methyl | 1 | 6-Chlor |
| 6 | n-Butyl | Methyl | 0 | -- |
| 7 | n-Propyl | Ethyl | 1 | 5-Chlor |
| 8 | n-Propyl | Ethyl | 1 | 6-Chlor |
| 9 | n-Propyl | Ethyl | 0 | -- |
| 10 | n-Butyl | Ethyl | 1 | 5-Chlor |
| 11 | n-Butyl | Ethyl | 1 | 6-Chlor |
| 12 | n-Butyl | Ethyl | 0 | -- |
| 13 | n-Propyl | n-Propyl | 1 | 5-Chlor |
| 14 | n-Propyl | n-Propyl | 1 | 6-Chlor |
| 15 | n-Propyl | n-Propyl | 0 | -- |
| 16 | n-Butyl | n-Propyl | 1 | 5-Chlor |
| 17 | n-Butyl | n-Propyl | 1 | 6-Chlor |
| 18 | n-Butyl | n-Propyl | 0 | -- |

Beispiele für bevorzugte Verbindungen I sind weiterhin die Verbindungen der allgemeinen Formeln I-A2, worin die Variablen R¹, R² und R³ jeweils die in einer Zeile der Tabelle 2 angegebenen Bedeutungen haben (Verbindungen I-A2.1 bis I-A2.18).

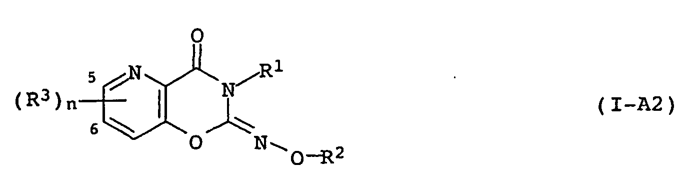

Beispiele für bevorzugte Verbindungen I sind weiterhin die Verbindungen der allgemeinen Formeln I-A3, worin die Variablen R¹, R² und R³ jeweils die in einer Zeile der Tabelle 2 angegebenen Bedeutungen haben (Verbindungen I-A3.1 bis I-A3.18).

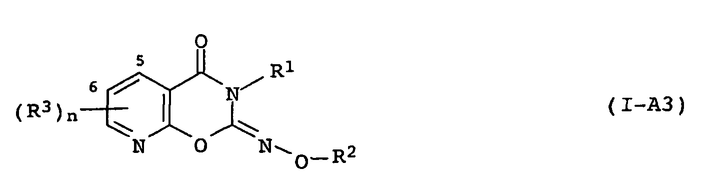

Beispiele für bevorzugte Verbindungen I sind weiterhin die Verbindungen der allgemeinen Formeln I-A4, worin die Variablen R¹, R² und R³ jeweils die in einer Zeile der Tabelle 2 angegebenen Bedeutungen haben (Verbindungen I-A4.1 bis I-A4.18).

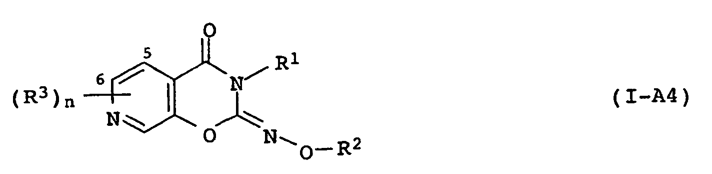

Beispiele für bevorzugte Verbindungen I sind weiterhin die Verbindungen der allgemeinen Formeln I-A5, worin die Variablen R¹, R² und R³ jeweils die in einer Zeile der Tabelle 2 angegebenen Bedeutungen haben (Verbindungen I-A5.1 bis I-A5.18).

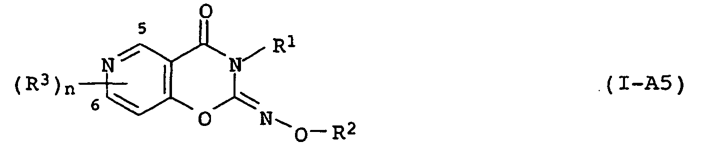

Weitere Beispiele für erfindungsgemäße Verbindungen I sind die Verbindungen der allgemeinen Formeln I-A6, I-A9, I-A10, I-A11, I-A12, I-A14, I-A15, I-A17, I-A18, I-A20 bis I-A27, I-A31, I-A33, I-A36, I-A37, worin die Variablen R¹, R² und R' bzw. R" jeweils die in einer der Zeilen 1 bis 27 bzw. 19 bis 36 der Tabelle 3 angegebenen Bedeutungen haben.

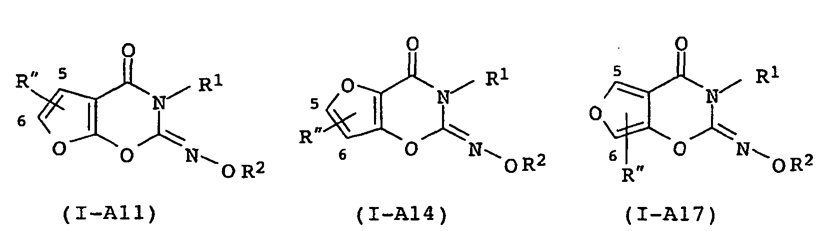

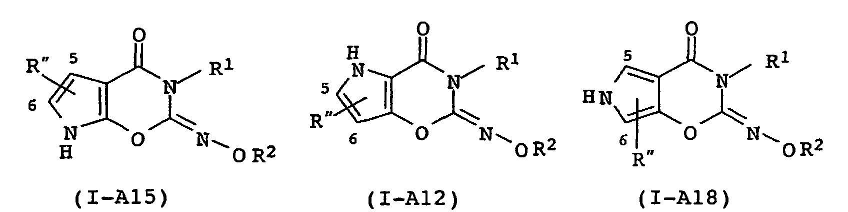

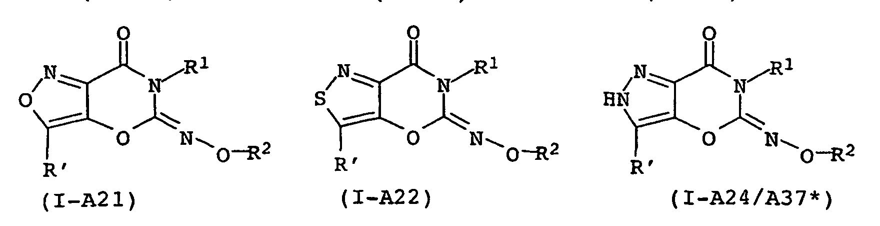

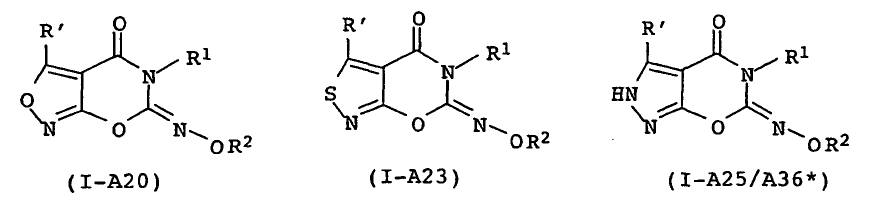

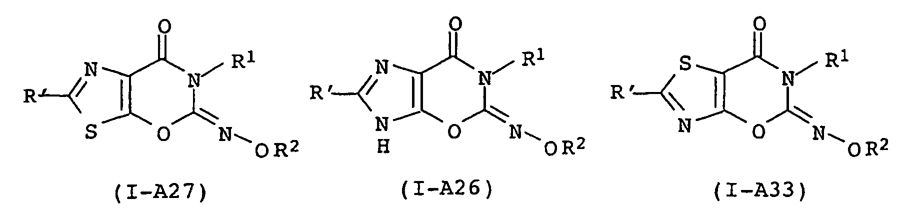

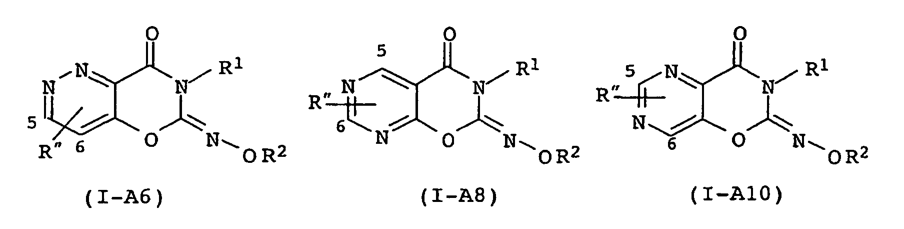

**Tabelle 3:**

| | R¹ | R² | n | R' | R" |
|---|---|---|---|---|---|
| 1 | n-Propyl | Methyl | 1 | -- | 5-Chlor |
| 2 | n-Propyl | Methyl | 1 | -- | 6-Chlor |
| 3 | n-Butyl | Methyl | 1 | -- | 5-Chlor |
| 4 | n-Butyl | Methyl | 1 | -- | 6-Chlor |
| 5 | n-Propyl | Ethyl | 1 | -- | 5-Chlor |
| 6 | n-Propyl | Ethyl | 1 | -- | 6-Chlor |
| 7 | n-Butyl | Ethyl | 1 | -- | 5-Chlor |
| 8 | n-Butyl | Ethyl | 1 | -- | 6-Chlor |
| 9 | n-Propyl | n-Propyl | 1 | -- | 5-Chlor |
| 10 | n-Propyl | n-Propyl | 1 | -- | 6-Chlor |
| 11 | n-Butyl | n-Propyl | 1 | -- | 5-Chlor |
| 12 | n-Butyl | n-Propyl | 1 | -- | 6-Chlor |
| 13 | Cyclopropylmethyl | Methyl | 1 | -- | 5-Chlor |
| 14 | Cyclopropylmethyl | Methyl | 1 | -- | 6-Chlor |
| 15 | Cyclopropylmethyl | Ethyl | 1 | -- | 5-Chlor |
| 16 | Cyclopropylmethyl | Ethyl | 1 | -- | 6-Chlor |
| 17 | Cyclopropylmethyl | n-Propyl | 1 | -- | 5-Chlor |
| 18 | Cyclopropylmethyl | n-Propyl | 1 | -- | 6-Chlor |
| 19 | n-Propyl | Methyl | 0 | H | |
| 20 | n-Butyl | Methyl | 0 | H | |
| 21 | n-Propyl | Ethyl | 0 | H | |
| 22 | n-Butyl | Ethyl | 0 | H | |
| 23 | n-Propyl | n-Propyl | 0 | H | |
| 24 | n-Butyl | n-Propyl | 0 | H | |
| 25 | Cyclopropylmethyl | Methyl | 0 | H | |
| 26 | Cyclopropylmethyl | Ethyl | 0 | H | |
| 27 | Cyclopropylmethyl | n-Propyl | 0 | H | |
| 28 | n-Propyl | Methyl | 1 | Chlor | -- |
| 29 | n-Butyl | Methyl | 1 | Chlor | -- |
| 30 | Cyclopropylmethyl | Methyl | 1 | Chlor | -- |
| 31 | n-Propyl | Ethyl | 1 | Chlor | -- |
| 32 | n-Butyl | Ethyl | 1 | Chlor | -- |

| | R¹ | R² | n | R' | R" |
|---|---|---|---|---|---|
| 33 | Cyclopropylmethyl | n-Propyl | 1 | Chlor | -- |
| 34 | n-Propyl | n-Propyl | 1 | Chlor | -- |
| 35 | n-Butyl | n-Propyl | 1 | Chlor | -- |
| 36 | Cyclopropylmethyl | n-Propyl | 1 | Chlor | -- |

Die Herstellung der Oxazinonverbindungen der Formel I kann analog zu einem in der Literatur beschriebenen Verfahren erfolgen (siehe Chemische Berichte Bd. 97, S. 3012 (1964), Bd. 98, S. 144 (1965)), das in Schema 1 dargestellt ist. In Schema 1 haben die Variablen R¹, R², R³, n und A die zuvor genannten Bedeutungen. Y steht für eine nucleophil verdrängbare Abgangsgruppe.

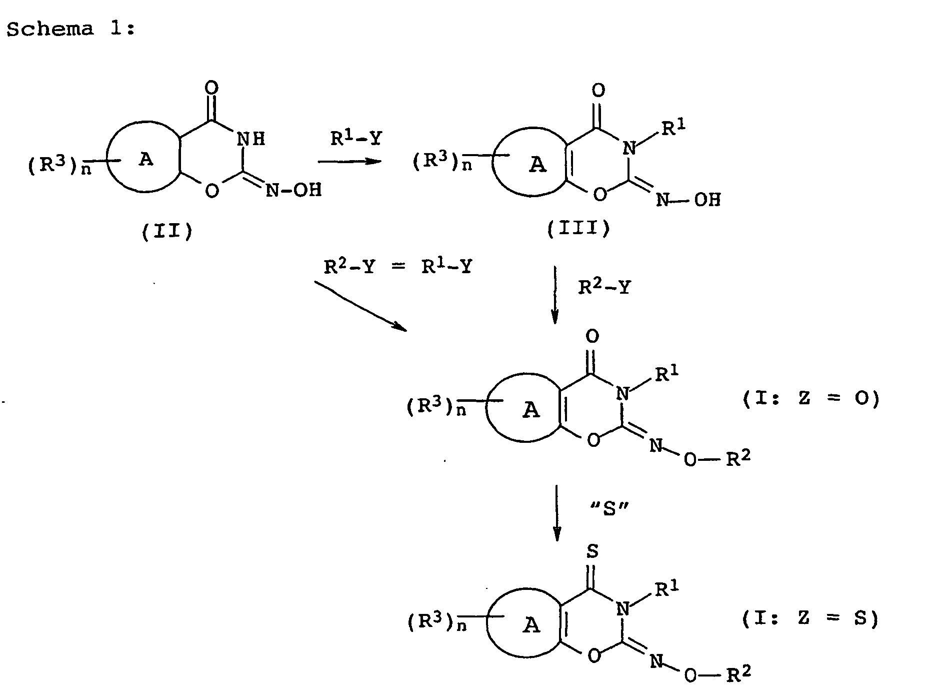

Hierzu werden die 2-Hydroxyiminoxazinone der allgemeinen Formel II sukzessive zu den Verbindungen I alkyliert. Wenn R¹ und R² gleich sind, kann die Alkylierung in einem Schritt erfolgen. Andernfalls erfolgt die Alkylierung in zwei aufeinanderfolgenden Schritten. Üblicherweise erfolgt zunächst durch Umsetzung von II mit einem Alkylierungsmittel R¹-Y die Alkylierung am Ringstickstoffatom von II, wohingegen das Sauerstoffatom der Hydroximinogruppe danach angegriffen wird (in Schema 1 dargestellt). Je nach Substraten und Reaktionsbedingungen kann sich die Reihenfolge aber auch umkehren, so dass man zunächst die Oxazinonverbindungen I mit R¹ = H und Z = O erhält, die man anschließend mit einem Alkylierungsmittel R¹-Y zur Verbindung I mit R¹ ≠ H und Z = O alkyliert (in Schema 1 nicht dargestellt). Der Fachmann kann durch einfache orientierende Versuche leicht feststellen, welche Alkylierungsabfolge zur Herstellung der Verbindung I geeigneter ist.

Als Alkylierungsmittel kommen Verbindungen in Betracht, in denen die Reste R¹ bzw. R² an eine geeignete, d.h. nucleophil verdrängbare Abgangsgruppe gebunden sind. Übliche Abgangsgruppen sind beispielsweise folgende Reste: Chlor, Brom, Iod, Methylsulfonyloxy, Phenylsulfonyloxy, Toluolsulfonyloxy, Trifluormethylsulfonyloxy. In der Regel setzt man das Alkylierungsmittel in wenigstens der stöchiometrisch erforderlichen, d. h. in äquimolarer Menge, vorzugsweise in einer Menge von 1 bis 2 Mol, je Mol Verbindung II bzw. III ein. Flüchtige Alkylierungsmitteln kann man auch in einem grösseren Überschuss einsetzen. Wenn R¹ und R² gleich sind und die Alkylierung des Ringstickstoffs und der Hydroxylfunktion gleichzeitig bewirkt werden soll setzt man entsprechend wenigstens 2 Mol Alkylierungsmittel je Mol Verbindung II ein.

Vorzugsweise führt man die Alkylierungen in Gegenwart einer Base durch. Als Base kommen grundsätzlich alle basischen Verbindungen in Betracht, die in der Lage sind, die Amidgruppe oder die Hydroxylgruppe der Hydroxyiminofunktion von II oder III zu deprotonieren. Hierzu zählen die Alkoholate, Amide, Hydride, Hydroxide, Carbonate und Hydrogencarbonate von Alkalimetallen oder Erdalkalimetallen, insbesondere des Lithiums, Kaliums, Natriums oder Kalziums. Beispiele hierfür sind die Natrium- oder Kaliumalkoholate des Methanols, des Ethanols, des n-Propanols, des iso-Propanols, des n-Butanols und des tert.-Butanols, weiterhin Natriumhydrid, Kalziumhydrid, Natriumamid, Kaliumamid, Natriumcarbonat, Kaliumcarbonat, Caesiumcarbonat, Kalziumcarbonat, Natriumhydrogencarbonat, Kaliumhydrogencarbonat, Natriumhydroxid, Kaliumhydroxid, Lithiumhydroxid, Lithiumdiisopropylamid. Geeignet sind weiterhin tertiäre Amine wie Triethylamin, Pyridin etc. Geeignet sind außerdem Organometallverbindungen des Lithiums wie Methyllithium, n-Butyllithium, n-Hexyllithium, Phenyllithium oder Grignardverbindungen z.B. des Methans, Ethans, Butans, Hexans, Cyclohexans oder Benzols. Vorzugsweise setzt man wenigstens eine äquimolare Menge an Base, bezogen auf die Verbindung II bzw. III ein. Insbesondere liegt das Molverhältnis von Base (gerechnet als Basenäquivalente) zu Verbindung II bzw. III im Bereich von 1:1 bis 1:5. Tertiäre Amine können auch in grösserem Überschuss eingesetzt werden, z.B. als Lösungsmittel oder Cosolvens.

Vorzugsweise führt man die Umsetzung in einem organischen Lösungsmittel durch. Als Lösungsmittel kommen alle bei Alkylierungen üblicherweise eingesetzten Lösungsmittel in Betracht, die Reaktanden bei der Reaktion in ausreichendem Maß lösen und sich selbst inert verhalten. Bevorzugt sind aprotisch polare Lösungsmittel, beispielsweise Ketone wie Aceton oder Methylethylketon, Acetonitril, Dimethylsulfoxid, Amide wie Dimethylformamid, Dimethylacetamid, N-Methylpyrrolidon und cyclische Harnstoffe, weiterhin aliphatische und alicyclische Ether wie Methyl-tert.-butylether, Diisopropylether, Dimethoxyethan, Diglykoldimethylether, Dioxan und Tetrahydrofuran sowie Aromaten wie Toluol, Xylol oder Chlorbenzol und Mischungen dieser Lösungsmittel.

Die für die Alkylierung erforderliche Temperatur kann je nach Reaktivität der Substrate und Alkylierungsmittel im Bereich von -80 bis +150°C liegen. Vorzugsweise erfolgt die Alkylierung bei Temperaturen im Bereich von -20 bis + 110°C.

Die Aufarbeitung des Umsetzungsproduktes zur Gewinnung der Zielverbindung I kann nach den hierfür üblichen Methoden erfolgen. In der Regel wird man zunächst extraktiv aufarbeiten oder das verwendete Lösungsmittel nach üblichen Verfahren, beispielsweise destillativ, entfernen. Man kann auch die Zielverbindung I aus der Reaktionsmischung nach Verdünnen der Reaktionsmischung mit Wasser mit einem flüchtigen organischen Lösungsmittel extrahieren, das seinerseits wieder destillativ entfernt wird. Auch kann man die Zielverbindung durch Zugabe von Wasser aus der Reaktionsmischung ausfällen. Hierbei erhält man ein Rohprodukt, welches das Wertprodukt I enthält. Zur weiteren Reinigung kann man die üblichen Verfahren wie Kristallisation oder Chromatographie, beispielsweise an Aluminiumoxiden oder Kieselgelen, einsetzen. Ebenfalls ist es möglich die nach dem Verfahren erhältlichen Stoffe an optisch aktiven Adsorbaten zur Gewinnung reiner Enantiomere zu chromatographieren.

Im Anschluss an die Alkylierung kann man die Ketogruppe in I mit üblichen Schwefelungsmitteln "S", in die Thiocarbonylfunktion umwandeln, wobei man Oxazinthionverbindungen I mit Z = S erhält.

Hierzu setzt man die Verbindungen I mit einem üblichen Schwefelungsmittel, z.B. P₂S₅ oder mit Lawesson's Reagenz [2,4-Bis(4-methoxyphenyl)-1,3-dithia-2,4-diphosphetan-2,4-disulfid], um. Die Umsetzung erfolgt vorzugsweise in einem inerten organischen Lösungsmittel, beispielsweise einem der vorgenannten Ether oder Aromaten oder Mischungen davon bei Temperaturen im Bereich von 0 bis 150°C. Entsprechende Verfahren sind aus der US 3,755,582 bekannt, auf die hiermit Bezug genommen wird.

Die Ausgangsverbindungen II können analog der oben angegebenen Literatur auf die in Schema 2 dargestellte Weise ausgehend von α-bzw. ortho-Hydroxycarbonsäureestern der Formel IV hergestellt werden. Verbindung II steht im Gleichgewicht mit seinem Tautomer IV, was für die folgende Alkylierung nur von untergeordneter Bedeutung ist. In den Formeln IV und V steht R z.B. für eine C₁-C₄-Alkylgruppe, insbesondere für eine Methylgruppe.

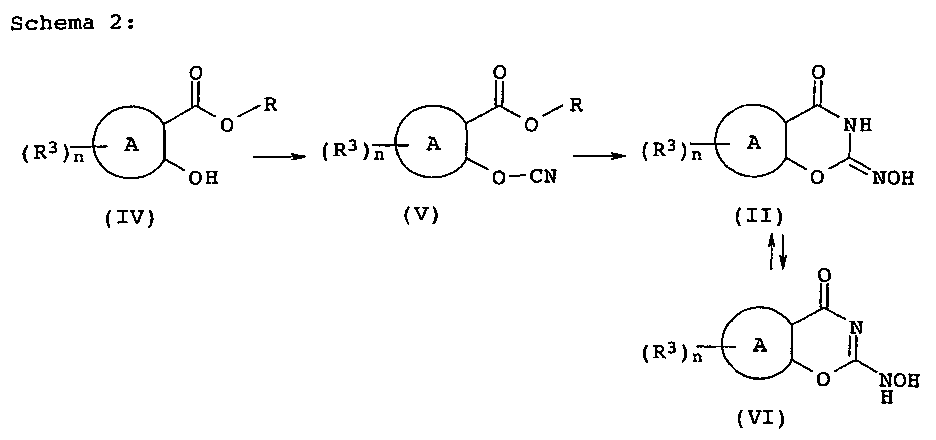

In einem ersten Schritt wird ein α- bzw. ortho-Hydroxycarbonsäureester der Formel IV mit einem Cyanierungsmittel wie Bromcyan oder Chlorcyan zum Cyanat V umgesetzt. Das Cyanierungsmittel wird in der Regel stöchiometrisch, d.h. äquimolar bezogen auf IV, eingesetzt, wobei Abweichungen von der exakten Stöchiometrie möglich sind, vorzugsweise aber nicht mehr als 20 Mol-% betragen. Die Cyanierung gelingt in der Regel bei Temperaturen im Bereich von -80 bis +100°C und vorzugsweise im Bereich von -40 bis + 60°C. Vorzugsweise führt man die Umsetzung in Gegenwart einer Hilfsbase durch, wobei als Hilfsbasen die bei der Alkylierung genannten Basen in Betracht kommen. Bevorzugte Basen sind die tertiären Amine. Die Base wird vorzugsweise äquimolar, bezogen auf IV eingesetzt, wobei Abweichungen von der exakten Stöchiometrie möglich sind.

Die Cyanierung von IV erfolgt in der Regel in einem organischen Lösungsmittel. Als organische Lösungsmittel kommen grundsätzlich alle in der Technik gebräuchlichen Lösungsmittel in Betracht, welche die Reaktanden bei der Reaktion in ausreichendem Maß lösen und sich selbst inert verhalten. Hierzu zählen insbesondere aprotisch polare Lösungsmittel, z.B. Ketone wie Aceton oder Methylethylketon, Nitrile wie Acetonitril, Dimethylsulfoxid, Amide wie Dimethylformamid, Dimethylacetamid oder N-Methylpyrrolidon, cyclische Harnstoffe, aliphatische und alicyclische Ether wie Diethylether, Diisopropylether, Methyl-tert.-butylether, Dimethoxyethan, Diglykoldimethylether, Dioxan oder Tetrahydrofuran sowie aromatische Kohlenwasserstoffe wie Toluol, Xylole oder Chlorbenzol und Mischungen dieser Lösungsmittel.

Der Ringschluss zu dem 2-Hydroxyiminooxazinon II erfolgt durch Umsetzung von V mit Hydroxylamin bzw. einem gebräuchlichen Salz z. B. dem Chlorid oder Sulfat des Hydroxylamins. Hydroxylamin wird dabei vorzugsweise in stöchiometrischer, d. h. wenigstens äquimolarer Menge eingesetzt, wobei auch ein Überschuss an Hydroxylamin eingesetzt werden kann, der vorzugsweise jedoch nicht mehr als 50 Mol-%, bezogen auf die Stöchiometrie der Reaktion, beträgt. Die Reaktionstemperaturen liegen in der Regel im Bereich von -20 bis +150°C und bevorzugt im Bereich von +20 bis 110°C.

Die Cyclisierung von V erfolgt üblicherweise in einem organischen Lösungsmittel, z.B. einem der vorgenannten Lösungsmittel, oder einer Mischung davon mit Wasser. Bevorzugte Lösungsmittel sind Alkohole, insbesondere C₁-C₃-Alkanole wie Methanol, Ethanol, n- oder i-Propanol und deren Mischungen sowie deren Mischungen mit Wasser. Es kann von Vorteil sein, das Lösungsmittel im Verlauf der Reaktion zu wechseln. So hat es sich bewährt, zunächst die Umsetzung in einem organischen Lösungsmittel, vorzugsweise einem der vorgenannten Alkohole zu beginnen und anschließend in Wasser die Reaktion abzuschließen.

Gelegentlich kann der Umsatz dadurch gefördert werden, dass man im Verlauf der Reaktion eine Base zugibt. Hierfür kommen unter anderem die Carbonate, Hydrogencarbonate und Hydroxide von Alkalimetalle und Erdalkalimetallen, z.B. Natriumhydrogencarbonat, Natriumcarbonat, Natriumhydroxid, Kaliumhydroxid, Kaliumcarbonat, Calciumhydroxid und Calciumcarbonat, sowie tertiäre Amine wie Triethylamin oder Pyridin in Betracht.

Anstelle von Hydroxylamin oder seinen Salzen kann man wie in Schema 3 dargestellt auch O-alkylierte Hydroxylamine R²-O-NH₂ oder deren Salze, z.B. der Halogenide oder Sulfate, einsetzen und erhält dann die Oxazinone I mit R¹ = H. Die Umsetzungsbedingungen entsprechen den für die Umsetzung mit Hydroxylamin genannten Bedingungen.

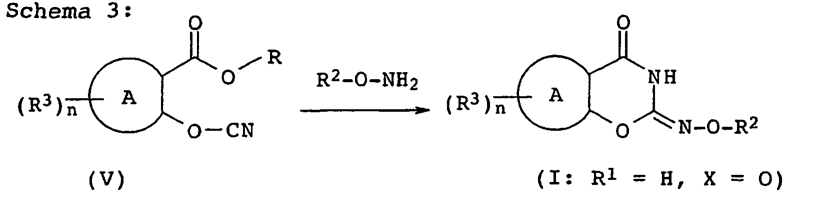

Die erfindungsgemäßen Verbindungen der Formel I können als Fungizide in ihrer Neutralform oder in Form eines Salzes eingesetzt werden, wobei sowohl Säureadditionssalze als auch Salze von Anionen der Verbindungen I mit den hierfür üblichen Kationen in Betracht kommen. Unter landwirtschaftlich brauchbaren Salzen kommen vor allem die Salze derjenigen Kationen oder die Säureadditionssalze derjenigen Säuren in Betracht, deren Kationen beziehungsweise Anionen die fungizide Wirkung der Verbindungen I nicht negativ beeinträchtigen. So kommen als Kationen insbesondere die Ionen der Alkalimetalle, vorzugsweise Natrium und Kalium, der Erdalkalimetalle, vorzugsweise Calcium, Magnesium und Barium, und der Übergangsmetalle, vorzugsweise Mangan, Kupfer, Zink und Eisen, sowie das Ammoniumion, das gewünschtenfalls ein bis vier C₁-C₄-Alkylsubstituenten und/oder einen Phenyl- oder Benzylsubstituenten tragen kann, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionssalzen sind in erster Linie Chlorid, Bromid, Fluorid, Hydrogensulfat, Sulfat, Dihydrogenphosphat, Hydrogenphosphat, Phosphat, Nitrat, Hydrogencarbonat, Carbonat, Hexafluorosilikat, Hexafluorophosphat, Benzoat, sowie die Anionen von C₁-C₄-Alkansäuren, vorzugsweise Formiat, Acetat, Propionat und Butyrat. Sie können durch Reaktion von I mit einer Säure des entsprechenden Anions, vorzugsweise der Chlorwasserstoffsäure, Bromwasserstoffsäure, Schwefelsäure, Phosphorsäure oder Salpetersäure, gebildet werden.

Die neuen Oxazin(thi)onverbindungen der Formel I und ihre Salze zeichnen sich durch eine hervorragende Wirksamkeit gegen ein breites Spektrum von pflanzenpathogenen Pilzen aus und können als Blatt- und Bodenfungizide eingesetzt werden. Sie besitzen zum Teil bemerkenswert hohe systemische Beweglichkeit und Wirksamkeit nach Boden- und insbesondere auch nach Blattapplikation.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- *Alternaria*-Arten an Gemüse und Obst,
- *Botrytis cinerea* (Grauschimmel) an Erdbeeren, Gemüse, Zierpflanzen und Reben,
- *Cercospora arachidicola* an Erdnüssen,
- *Erysiphe cichoracearum* und *Sphaerotheca fuliginea* an Kürbisgewächsen,
- *Erysiphe graminis* (echter Mehltau) an Getreide,
- *Fusarium-* und *Verticillium*-Arten an verschiedenen Pflanzen,
- *Helminthosporium*-Arten an Getreide,
- *Mycosphaerella*-Arten an Bananen und Erdnüssen,
- *Phytophthora infestans* an Kartoffeln und Tomaten,
- *Plasmopara viticola* an Reben,
- *Podosphaera leucotricha* an Äpfeln,
- *Pseudocercosporella herpotrichoides* an Weizen und Gerste,
- *Pseudoperonospora*-Arten an Hopfen und Gurken,
- *Puccinia*-Arten an Getreide,
- *Pyricularia oryzae* an Reis,
- *Rhizoctonia*-Arten an Baumwolle, Reis und Rasen,
- *Septoria nodorum* an Weizen,
- *Sphaerotheca fuliginea* (Gurkenmehltau) an Gurken,
- *Uncinula necator* an Reben,
- *Ustilago*-Arten an Getreide und Zuckerrohr, sowie
- *Venturia*-Arten (Schorf) an Äpfeln und Birnen.

Die Verbindungen I eignen sich außerdem zur Bekämpfung von Schadpilzen wie *Paecilomyces variotii* im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Kubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wässrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.-% des Wirkstoffs enthält.
IX. 10 Gew.-Teile der erfindungsgemäßen Verbindung werden in 63 Gew.-Teilen Cyclohexanon, 27 Gew.-Teilen Dispergiermittel (beispielsweise eine Mischung aus 50 Gew.-Teilen des Anlagerungsprodukts von 7 Mol Ethylenoxid an 1 Mol Isooctylphenol und 50 Gew.-Teilen des Anlagerungsprodukts von 40 Mol Ethylenoxid an 1 Mol Ricinusöl) gelöst. Die Stammlösung wird anschließend durch Verteilen in Wasser auf die gewünschte Konzentration verdünnt, z.B. auf eine Konzentration im Bereich von 1 bis 100 ppm.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%. Häufig reichen bereits geringe Wirkstoffmengen an Verbindung I in der anwendungsfertigen Zubereitung aus, z.B. 2 bis 200 ppm. Ebenso sind anwendungsfertige Zubereitungen mit Wirkstoffkonzentrationen im Bereich von 0,01 bis 1 % bevorzugt.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol; α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[a-(o-tolyloxy)-o-tolyl]acetat, Methyl-E-2-{2-[6-(2-cyanophenoxy)-pyrimidin-4-yl-oxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid, Methyl-E-2-{2-[(2-trifluormethylpyridyl-6-)oxymethyl]-phenyl}-3-methoxyacrylat, (E,E)-Methoximino-{2-[1-(3-trifluormethylphenyl)-ethylidenaminooxymethyl]-phenyl}-essigsäuremethylester, Methyl-N-(2-{[1-(4-chlorphenyl)-1H-pyrazol-3-yl]oxymethyl}phenyl)-N-methoxycarbamat,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid,
- sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die folgenden Beispiele sollen die Erfindung erläutern und sind nicht einschränkend zu verstehen.

### Herstellungsbeispiele:

### Beispiel 1: O-Methyl-(6-Chlor-3-propyl-4H-1,3-benz[e]oxazin-4(3H)-on)-2-oxim (Verbindung I-A1.2)

### 1.1 2-Cyanato-5-chlorbenzoesäuremethylester

Zu 21,0 g (0,198 Mol) Bromcyan in 200 ml Aceton gab man bei -20°C unter Rühren 20,0 g (0,198 Mol) Triethylamin. Nach 5 min. gab man hierzu 36,9 g (0,198 Mol) 5-Chlor-2-hydroxybenzoesäuremethylester und rührte 30 min. bei -20°C nach. Man filtrierte, wusch den Filter mit 200 ml Aceton nach und engte die vereinigten organischen Phasen im Vakuum ein. Man erhielt 34,4 g der Titelverbindung als gelben Feststoff, der direkt weiter umgesetzt wurde.

### 1.2 6-Chlor-4H-1,3-benz[e]oxazin-4(3H)-on-2-oxim

34,4 g (0,163 Mol) 2-Cyanato-5-chlorobenzoesäuremethylester in 400 ml Methanol wurden mit 11,3 g (0,163 Mol) Hydroxylaminhydrochlorid 1 h bei Raumtemperatur gerührt. Anschließend wurde das Lösungsmittel im Vakuum entfernt und der Rückstand wurde mit Diethylether gewaschen. Nach Zugabe einer Lösung von 20,5 g Natriumhydrogencarbonat in 400 ml Wasser wurde das Rohprodukt 16 h gerührt, abfiltriert, nochmals 16 h mit 500 ml Wasser gerührt, abfiltriert und getrocknet. Man erhielt so 12,3 g 6-Chlor-4H-1,3-benz[e]oxazin-4(3H)-on-2-oxim.

### 1.3 6-Chlor-3-propyl-4H-1,3-benz[e]oxazin-4(3H)-on-2-oxim

Eine Lösung von 5,0 g (23,5 mmol) 6-Chlor-4H-1,3-benz[e]oxazin-4(3H)-on-2-oxim in 100 ml Dimethylformamid wurden bei Raumtemperatur unter Rühren mit 0,94 g (23,5 mmol) 60%igem Natriumhydrid versetzt. Anschließend gab man 4,3 g (35 mmol) Brompropan zu und rührte 16 h bei Raumtemperatur nach. Man gab die so erhaltene Mischung auf 100 ml wässrige Natriumdihydrogenphosphat-Lösung (10%ig) und extrahierte viermal mit je 100 ml Methyl-tert.-butylether. Die vereinigten Extrakte wurden mit 100 ml gesättigter Kochsalzlösung gewaschen, über Natriumsulfat getrocknet und im Vakuum eingeengt. Der Rückstand wurde durch Chromatographie an Kieselgel mit Cyclohexan/Essigsäureethylester gereinigt. Man erhielt so 1,8 g der Titelverbindung mit einem Schmelzpunkt von 144°C.

### 1.4 O-Methyl-(6-Chlor-3-propyl-4H-1,3-benz[e]oxazin-4(3H)-on)-2-oxim

Zu 80 mg (2,1 mmol) 60%igem Natriumhydrid in 10 ml Dimethylformamid gab man bei Raumtemperatur unter Rühren sofort eine Lösung von 500 mg (2,0 mmol) 6-Chlor-3-propyl-4H-1,3-benz[e]oxazin-4(3H)-on-2-oxim in 20 ml Dimethylformamid. Anschließend gab man hierzu 430 mg (3,0 mmol) Methyliodid und rührte 16 h bei Raumtemperatur nach. Die erhaltene Mischung gab man auf 50 ml wässrige Natriumdihydrogenphosphat-Lösung (10 %ig) und extrahierte viermal mit je 50 ml Methyl-tert.-butylether. Die vereinigten Extrakte wurden mit 30 ml gesättigter Kochsalzlösung gewaschen, über Natriumsulfat getrocknet und im Vakuum eingeengt. Hierbei fiel die Titelverbindung als Rohprodukt an das zur Reinigung nacheinander mit Diethylether und n-Hexan gewaschen wurde. Man erhielt so 230 mg der Titelverbindung mit einem Schmelzpunkt von 158°C.

### Beispiel 2: O-Methyl-(6-iod-3-propyl-4H-1,3-benz[e]oxazin-4(3H)-on)-2-oxim (Verbindung I-A1.4)

### 2.1 O-Methyl-(6-iod-4H-1,3-benz[e]oxazin-4(3H)-on)-2-oxim

2,0 g (6,6 mmol) 2-Cyanato-5-iod-benzoesäuremethylester, 0,55 g (6,6 mmol) O-Methylhydroxylaminhydrochlorid und 0,55 g (6,6 mmol) Natriumhydrogencarbonat wurden in 20 ml Methanol 1 h bei Raumtemperatur gerührt. Anschließend wurde das Lösungsmittel im Vakuum entfernt und der Rückstand wurde in 10 ml 10 gew.-%ige Natronlauge gegeben. Nach 5 min stellte man mit 10 gew.-%iger Salzsäure einen pH-Wert von 6-8 ein. Das ausgefallene Produkt wurde abfiltriert, gewaschen und im Vakuum getrocknet. Ausbeute 2,08 g weißer Feststoff. ¹H-NMR (d₆-DMSO, es sind 2 Isomere zu beobachten) δ = 3,70 (s); 7,12 und 7,18 (d); 8,00 (m); 8,05 (s); 11,35 und 11,70 (s).

### 2.2 O-Methyl-(6-iod-3-propyl-4H-1,3-benz[e]oxazin-4(3H)-on)-2-oxim

2,94 g (9,2 mmol) O-Methyl-(6-iod-4H-1,3-benz[e]oxazin-4(3H)-on)-2-oxim in 50 ml Dimethylsulfoxid wurden bei Raumtemperatur unter Rühren mit 1,24 g (1,1 mmol) Kalium-tert.-butylat versetzt und 5 min nachgerührt, wobei die Temperatur auf 30°C anstieg. Anschließend gab man 1,40 g (1,1 mmol) Brompropan zu und rührte 16 h bei Raumtemperatur nach. Die Mischung wurde auf 100 ml wässrige Natriumdihydrogenphosphat-Lösung gegeben und dreimal mit je 100 ml Methyl-tert.-butylether extrahiert. Die vereinigten Extrakte wurden einmal mit 30 ml Natriumdihydrogenphosphat-Lösung und einmal mit 30 ml gesättigter Kochsalzlösung gewaschen, über Natriumsulfat getrocknet und im Vakuum eingeengt. Ausbeute 2,2 g weißer Feststoff. ¹H-NMR (CDCl₃) δ = 0,95 (t); 1,75 (m); 3,90 (s); 3,93 (q); 7,00 (d); 7,87 (d); 8,32 (s).

In analoger Weise wurden die in der folgenden Tabelle 4 angegebenen Wirkstoffe der allgemeinen Formel I-A1 hergestellt.

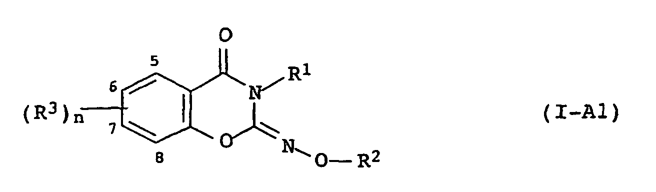

**Tabelle 4:**

| Nr. | R¹ | R² | n | R³ | Physikalische Daten |
|---|---|---|---|---|---|
| I-A1.4 | n-Propyl | Methyl | 1 | 6-Iod | 126°C |
| I-A1.8 | n-Propyl | Ethyl | 1 | 6-Iod | 143°C |
| I-A1.10 | n-Propyl | n-Propyl | 1 | 6-Chlor | 79°C |
| I-A1.12 | n-Propyl | n-Propyl | 1 | 6-Iod | 120°C |
| I-A1.20 | n-Butyl | Methyl | 1 | 6-Iod | 113°C |
| I-A1.24 | n-Butyl | Ethyl | 1 | 6-Iod | 137°C |
| I-A1.28 | n-Butyl | n-Propyl | 1 | 6-Iod | 105°C |
| I-A1.68 | Cyclopropylmethyl | Methyl | 1 | 6-Iod | 141°C |
| I-A1.72 | Cyclopropylmethyl | Ethyl | 1 | 6-Iod | 119°C |
| I-A1.76 | Cyclopropylmethyl | n-Propyl | 1 | 6-Iod | ¹H-NMR (CDCl₃) δ = 0,48 (m); 0,97 (t); 1,36 (m); 1,75 (m); 3,87 (d); 4,00 (t); 7,04 (d); 7,85 (d); 8,32 (s). |
| 1-A1.129 | n-Propyl | n-Propyl | 0 | -- | 52°C |
| 1-A1.130 | n-Propyl | n-Propyl | 1 | 6-Methyl | ¹H-NMR (d₆-DMSO) d= 0,93 (t); 1,03 (t); 1,68 (m); 2,40 (s); 3,75 (t); 4,02 (t); 7,35 (d); 7,55 (d); 7,70 (s) |
| 1-A1.131 | Methyl | n-Propyl | 1 | 6-Methyl | 50°C |
| 1-A1.132 | n-Propyl | Methyl | 1 | 6-Methyl | 125°C |
| 1-A1.133 | n-Propyl | Methyl | 1 | 6-Methoxy | 107°C |
| 1-A1.134 | Cyclopropylmethyl | 2-Propyl | 1 | 6-Iod | 88°C |
| 1-A1.135 | Cyclopropylmethyl | Cyclopropylmethyl | 1 | 6-Iod | 142°C |
| 1-A1.136 | n-Butyl | Cyclopropylmethyl | 1 | 6-Iod | 121°C |
| 1-A1.137 | n-Propyl | Cyclopropylmethyl | 1 | 6-Iod | 155°C |
| 1-A1.138 | n-Butyl | 2-Propyl | 1 | 6-Iod | 89°C |
| 1-A1.139 | n-Propyl | 2-Propyl | 1 | 6-Iod | 90°C |

### Anwendungsbeispiele

### Beispiele für die Wirkung gegen Schadpilze

Die fungizide Wirkung der Verbindungen der allgemeinen Formel I ließ sich durch die folgenden Versuche zeigen:

Die Wirkstoffe wurden als wässrige Wirkstoffaufbereitungen eingesetzt, die den Wirkstoff in einer in Konzentrationen von 250 ppm, 63 ppm, 16 ppm bzw. 4 ppm enthielten. Die wässrige Wirkstoffaufbereitung wurde durch verdünnen einer Stammlösung aus 10 Gew.-% Wirkstoff, 63 Gew.-% Cyclohexanon und 27 Gew.-% Emulgiermittel (20 Gew.-Teile Nekanil® LN (Lutensol® AP6, Netzmittel mit Emulgier- und Dispergierwirkung auf der Basis ethoxylierter Alkylphenole) und 10 Gew.-Teile Wettol® EM (nichtionischer Emulgator auf der Basis von ethoxyliertem Ricinusöl)) mit Wasser in der für die gewünschte Konzentration erforderlichen Menge hergestellt.

### Anwendungsbeispiel 1 - protektive Wirksamkeit gegen Gurkenmehltau

Blätter von in Töpfen gewachsenen Gurkenkeimlingen der Sorte "Chinesische Schlange" wurden im Keimblattstadium mit wässriger Wirkstoffaufbereitung bis zur Tropfnässe besprüht. 20 Stunden nach dem Antrocknen des Spritzbelages wurden die Pflanzen mit einer wässrigen Sporensuspension des Gurkenmehltaus (*Sphaerotheca fuliginea*) inokuliert. Anschließend wurden die Pflanzen im Gewächshaus bei Temperaturen zwischen 20 und 24°C und 60 bis 80 % relativer Luftfeuchtigkeit für 7 Tage kultiviert. Dann wurde das Ausmaß der Mehltauentwicklung visuell in %-Befall der Keimblattfläche ermittelt.

In diesem Versuch zeigten bereits die mit 4 ppm des Wirkstoffs aus Beispiel 1 (Verbindung I-A1.2 aus Tabelle 1) behandelten Pflanzen keinen Befall während die unbehandelten Pflanzen zu 90 % befallen waren. Höhere Aufwandmengen führten zu dem gleichen Ergebnis wie bei 4 ppm. Im übrigen führte die Anwendung von anderen in Tabelle 4 angegebenen Verbindungen zu den folgenden in Tabelle 5 angegebenen Ergebnissen.

**Tabelle 5:**

| Wirkstoff | %-Befall der Blätter bei der angegebenen Wirkstoffkonzentration in der wäßriger Wirkstoffaufbereitung | | |
|---|---|---|---|
| | 250 ppm | 63 ppm | 16 ppm |
| Verbindung I - A1.2 | 0 | 0 | 0 |
| Verbindung I - A1.4 | 0 | 0 | 0 |
| Verbindung I - A1.8 | 0 | 0 | 0 |
| Verbindung I - A1.12 | 0 | 0 | 0 |
| Verbindung I - A1.20 | 0 | 0 | 0 |
| Verbindung I - A1.24 | 0 | 0 | 0 |
| Verbindung I - A1.28 | 0 | 0 | 0 |
| Verbindung I - A1.68 | 0 | 0 | 0 |
| Verbindung I - A1.72 | 0 | 0 | 0 |
| Verbindung I - A1.76 | 0 | 0 | 0 |
| Verbindung I - A1.132 | 0 | 10 | 10 |
| Verbindung I - A1.133 | 0 | 5 | 10 |
| Verbindung I - A1.134 | 0 | 0 | 0 |
| Verbindung I - A1.135 | 0 | 0 | 3 |
| Verbindung I - A1.136 | 0 | 0 | 0 |
| Verbindung I - A1.137 | 0 | 0 | 0 |
| Verbindung I - A1.138 | 0 | 0 | 0 |
| Verbindung I - A1.139 | 0 | 0 | 0 |
| Unbehandelt | 90 | | |

### Anwendungsbeispiel 2 - protektive Wirksamkeit gegen Weizenmehltau verursacht durch Erysiphe [syn. Blumeria] graminis forma specialis tritici

Die ersten vollständig entwickelten Blätter von in Töpfen gewachsenen Weizenkeimlingen der Sorte "Kanzler" wurden mit wäßriger Wirkstoffaufbereitung, die aus einer Stammlösung bestehend aus 10 % Wirkstoff, 85 % Cyclohexanon und 5 % Emulgiermittel angesetzt wurde, bis zur Tropfnässe besprüht und 24 Stunden nach dem Antrocknen des Spritzbelages mit Sporen des Weizenmehltaus (*Erysiphe [syn. Blumeria] graminis* forma specialis. *tritici*) bestäubt. Die Versuchspflanzen wurden anschließend im Gewächshaus bei Temperaturen zwischen 20 und 24° C und 60 bis 90 % relativer Luftfeuchtigkeit aufgestellt. Nach 7 Tagen wurde das Ausmaß der Mehltauentwicklung visuell in % Befall der gesamten Blattfläche ermittelt. Die Ergebnisse sind in Tabelle 6 zusammengestellt.

**Tabelle 6:**

| Wirkstoff | %-Befall der Blätter bei der angegebenen wirkstoffkonzentration in der wäßriger Wirkstoffaufbereitung | | |
|---|---|---|---|
| | 250 ppm | 63 ppm | 16 ppm |
| Verbindung I - A1.2 | 0 | 0 | 0 |
| Verbindung I - A1.4 | 0 | 0 | 0 |
| Verbindung I - A1.8 | 0 | 0 | 0 |
| Verbindung I - A1.12 | 0 | 0 | 0 |
| Verbindung I - A1.20 | 0 | 0 | 0 |
| Verbindung I - A1.24 | 0 | 0 | 0 |
| Verbindung I - A1.28 | 0 | 0 | 0 |
| Verbindung I - A1.68 | 0 | 0 | 0 |
| Verbindung I - A1.72 | 0 | 0 | 0 |
| Verbindung I - A1.76 | 0 | 0 | 0 |
| Verbindung I - A1.133 | 0 | 0 | 20 |
| Verbindung I - A1.134 | 0 | 0 | 0 |
| Verbindung I - A1.135 | 0 | 0 | 5 |
| Verbindung I - A1.136 | 0 | 0 | 0 |
| Verbindung I - A1.137 | 0 | 0 | 0 |
| Verbindung I - A1.138 | 0 | 0 | 0 |
| Verbindung I - A1.139 | 0 | 0 | 0 |
| Unbehandelt | 85 | | |

## Patentansprüche

1. Oxazin(thi)onverbindungen der allgemeinen Formel I 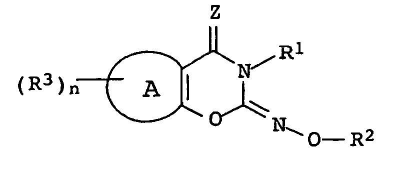 in der die Variablen Z, A, n, R¹, R² und R³ und n die folgenden Bedeutungen haben:
Z Sauerstoff oder Schwefel,
A ein über zwei C-Atome mit dem Oxazin(thi)onring anellierter 5- oder 6-gliedriger Carbocyclus oder ein 5- oder 6-gliedriger Heterocyclus mit 1, 2 oder 3 Heteroatomen, ausgewählt unter N, O und S;
n eine Zahl von 0 bis 4;
R¹ Wasserstoff, C₁-C₆-Alkyl, C₂-C₆-Alkenyl, C₂-C₆-Alkinyl, die jeweils gegebenenfalls durch O-R⁴, C(O)O-R⁵, NR⁶R⁷, C(O)NR⁶R⁷, S-R⁸, C₃-C₈-Cycloalkyl, Phenyl oder durch 5- oder 6-gliedriges, gesättigtes oder ungesättigtes Heterocyclyl mit 1 bis 3 Heteroatomen, ausgewählt unter N, O und S, substituiert sind, wobei Phenyl und Heterocyclyl gegebenenfalls ein-, zwei- oder dreifach substituiert sind, C₁-C₆-Halogenalkyl, C₂-C₆-Halogenalkenyl oder C₂-C₆-Halogenalkinyl;
R² C₁-C₃-Alkyl, C₁-C₃-Halogenalkyl oder Cyclopropylmethyl;
R³ Halogen, Cyano, C₁-C₆-Alkyl, C₁-C₆-Halogenalkyl, C₁-C₆-Alkoxy oder C₁-C₆-Halogenalkoxy;
und worin
R⁴, R⁵ unabhängig voneinander für Wasserstoff, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, Hydroxy-C₁-C₄-alkyl, Hydroxycarbonyl-C₁-C₄-alkyl, C₁-C₄-Alkoxy-C₁-C₄-alkyl oder C₁-C₄-Alkoxycarbonyl-C₁-C₄-alkyl;
R⁶, R⁷ unabhängig voneinander für Wasserstoff, C₁-C₆-Alkyl, C₁-C₆-Alkoxy, C₂-C₆-Alkenyl, C₂-C₆-Alkinyl, C₁-C₆-Halogenalkyl, C₁-C₆-Halogenalkoxy, C₂-C₆-Halogenalkenyl, C₂-C₆-Halogenalkinyl, Hydroxy-C₁-C₄-alkyl, Hydroxycarbonyl-C₁-C₄-alkyl, C₁-C₄-Alkoxy-C₁-C₄-alkyl oder C₁-C₄-Alkoxycarbonyl-C₁-C₄-alkyl;
R⁶ und R⁷ gemeinsam mit dem Stickstoffatom an das sie gebunden sind auch einen 5-, 6- oder 7-gliedrigen, gesättigten N-Heterocyclus bilden können, der ein weiteres Heteroatom, ausgewählt unter O, N und S aufweisen kann und/oder durch 1 bis 4 C₁-C₆-Alkylgruppen substituiert sein kann;
R⁸ für C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, Hydroxy-C₁-C₄-alkyl, Hydroxycarbonyl-C₁-C₄-alkyl, C₁-C₄-Alkoxy-C₁-C₄-alkyl oder C₁-C₄-Alkoxycarbonyl-C₁-C₄-alkyl stehen;
sowie die landwirtschaftlich brauchbaren Salze der Oxazin(thi)onverbindungen I.

2. Verbindungen nach Anspruch 1, worin A für einen anellierten Benzol-, Pyridin- oder Thiophen-Ring steht.

3. Verbindungen nach Anspruch 1 oder 2, worin R¹ für C₁-C₆-Alkyl, C₂-C₆-Alkenyl, C₁-C₆-Halogenalkyl, C₂-C₆-Halogenalkenyl, C₁-C₄-Alkoxy-C₁-C₄-alkyl oder C₃-C₈-Cycloalkyl-C₁-C₄-alkyl.

4. Verbindungen nach einem der vorhergehenden Ansprüche, worin n für 1, 2, 3 oder 4 steht.

5. Verbindungen nach einem der vorhergehenden Ansprüche, worin R³ für C₁-C₄-Alkyl, C₁-C₄-Halogenalkyl, C₁-C₄-Halogenalkoxy oder Halogen steht.

6. Verbindungen nach einem der vorhergehenden Ansprüche, worin A für einen anellierten Benzolring steht und die Variablen Z, R¹, R², R³ und n die folgenden Bedeutungen haben:
Z Sauerstoff;
R¹ C₁-C₆-Alkyl, C₂-C₆-Alkenyl, C₁-C₆-Halogenalkyl, C₂-C₆-Halogenalkenyl, C₁-C₄-Alkoxy-C₁-C₄-alkyl oder C₃-C₈-Cycloalkyl-C₁-C₄-alkyl;
R² C₁-C₃-Alkyl, C₁-C₃-Halogenalkyl oder Cyclopropylmethyl;
R³ C₁-C₄-Alkyl, C₁-C₄-Halogenalkyl, C₁-C₄-Halogenalkoxy oder Halogen;
n für 1 oder 2.

7. Verbindungen nach einem der Ansprüche 1 bis 5, worin A für einen anellierten Thiophenring steht und die Variablen Z, R¹, R², R³ und n die folgenden Bedeutungen haben:
Z Sauerstoff;
R¹ C₁-C₆-Alkyl, C₂-C₆-Alkenyl, C₁-C₆-Halogenalkyl, C₂-C₆-Halogenalkenyl, C₁-C₄-Alkoxy-C₁-C₄-alkyl oder C₃-C₈-Cycloalkyl-C₁-C₄-alkyl;
R² C₁-C₃-Alkyl, C₁-C₃-Halogenalkyl oder Cyclopropylmethyl;
R³ C₁-C₄-Alkyl, C₁-C₄-Halogenalkyl, C₁-C₄-Halogenalkoxy oder Halogen;
n für 1 oder 2.

8. Verwendung von Verbindungen der Formel I und deren landwirtschaftlich brauchbaren Salzen gemäß Anspruch 1 zur Bekämpfung von pflanzenpathogenen Pilzen.

9. Mittel, enthaltend eine fungizid wirksame Menge mindestens einer Verbindung der Formel I und/oder mindestens eines landwirtschaftlich brauchbaren Salzes von I gemäß Anspruch 1 sowie wenigstens einen Trägerstoff.

10. Verfahren zur Bekämpfung von pflanzenpathogenen Pilzen, wobei man die Pilze oder die von Pilzbefall bedrohten Materialien, Pflanzen, Saatgüter oder den Erdboden mit einer fungizid wirksamen Menge mindestens einer Verbindung der Formel I gemäß Anspruch 1 und/oder eines Salzes von I behandelt.

## Claims

1. An oxazin(ethi)one compound of the formula I 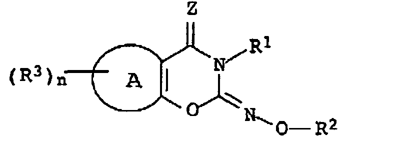 in which the variables Z, A, n, R¹, R² and R³ and n are as defined below:
Z is oxygen or sulfur,
A is a 5- or 6-membered carbocycle or a 5- or 6-membered heterocycle having 1, 2 or 3 heteroatoms selected from the group consisting of N, O and S, each of which cycles is fused to the oxazin(ethi)one ring via two carbon atoms;
n is a number from 0 to 4;
R¹ is hydrogen, is C₁-C₆-alkyl, C₂-C₆-alkenyl, C₂-C₆-alkynyl, which are in each case unsubstituted or substituted by O-R⁴, C(O)O-R⁵, NR⁶R⁷, C(O)NR⁶R⁷, S-R⁸, C₃-C₈-cycloalkyl, phenyl or by 5- or 6-membered saturated or unsaturated heterocyclyl having 1 to 3 heteroatoms selected from the group consisting of N, 0 and S, where phenyl and heterocyclyl may be mono-, di- or trisubstituted by C₁-C₆-haloalkyl, C₂-C₆-haloalkenyl or C₂-C₆-haloalkynyl;
R² is C₁-C₃-alkyl, C₁-C₃-haloalkyl or cyclopropylmethyl;
R³ is halogen, cyano, C₁-C₆-alkyl, C₁-C₆-haloalkyl, C₁-C₆-alkoxy or C₁-C₆-haloalkoxy;
and in which
R⁴, R⁵ independently of one another are hydrogen, C₁-C₆-alkyl, C₂-C₆-alkenyl, C₂-C₆-alkynyl, C₁-C₆-haloalkyl, C₂-C₆-haloalkenyl, C₂-C₆-haloalkynyl, hydroxy-C₁-C₄-alkyl, hydroxycarbonyl-C₁-C₄-alkyl, C₁-C₄-alkoxy-C₁-C₄-alkyl or C₁-C₄-alkoxycarbonyl-C₁-C₄-alkyl;
R⁶, R⁷ independently of one another are hydrogen, C₁-C₆-alkyl, C₁-C₆-alkoxy, C₂-C₆-alkenyl, C₂-C₆-alkynyl, C₁-C₆-haloalkyl, C₁-C₆-haloalkoxy, C₂-C₆-haloalkenyl, C₂-C₆-haloalkynyl, hydroxy-C₁-C₄-alkyl, hydroxycarbonyl-C₁-C₄-alkyl, C₁-C₄-alkoxy-C₁-C₄-alkyl or C₁-C₄-alkoxycarbonyl-C₁-C₄-alkyl;
R⁶ and R⁷ together with the nitrogen atom to which they are attached may also form a 5-, 6- or 7-membered saturated N-heterocycle which may contain a further heteroatom selected from the group consisting of 0, N and S and/or which may be substituted by 1 to 4 C₁-C₆-alkyl groups;
R⁸ represents C₁-C₆-alkyl, C₂-C₆-alkenyl, C₂-C₆-alkynyl, C₁-C₆-haloalkyl, C₂-C₆-haloalkenyl, C₂-C₆-haloalkynyl, hydroxy-C₁-C₄-alkyl, hydroxycarbonyl-C₁-C₄-alkyl, C₁-C₄-alkoxy-C₁-C₄-alkyl or C₁-C₄-alkoxycarbonyl-C₁-C₄-alkyl;
and the agriculturally useful salts of the oxazin(ethi)one compound I.

2. A compound as claimed in claim 1 where A is a fused benzene, pyridine or thiophene ring.

3. A compound as claimed in claim 1 or 2 where R¹ is C₁-C₆-alkyl, C₂-C₆-alkenyl, C₁-C₆-haloalkyl, C₂-C₆-haloalkenyl, C₁-C₄-alkoxy-C₁-C₄-alkyl or C₃-C₈-cycloalkyl-C₁-C₄-alkyl.

4. A compound as claimed in any of the preceding claims where n is 1, 2, 3 or 4.

5. A compound as claimed in any of the preceding claims where R³ is C₁-C₄-alkyl, C₁-C₄-haloalkyl, C₁-C₄-haloalkoxy or halogen.

6. A compound as claimed in any of the preceding claims where A is a fused benzene ring and the variables Z, R¹, R², R³ and n are as defined below:
Z is oxygen;
R¹ is C₁-C₆-alkyl, C₂-C₆-alkenyl, C₁-C₆-haloalkyl, C₂-C₆-haloalkenyl, C₁-C₄-alkoxy-C₁-C₄-alkyl or C₃-C₈-cycloalkyl-C₁-C₄-alkyl;
R² is C₁-C₃-alkyl, C₁-C₃-haloalkyl or cyclopropylmethyl;
R³ is C₁-C₄-alkyl, C₁-C₄-haloalkyl, C₁-C₄-haloalkoxy or halogen;
n is 1 or 2.

7. A compound as claimed in any of claims 1 to 5, where A is a fused thiophene ring and the variables Z, R¹, R², R³ and n are as defined below:
Z is oxygen;
R¹ is C₁-C₆-alkyl, C₂-C₆-alkenyl, C₁-C₆-haloalkyl, C₂-C₆-haloalkenyl, C₁-C₄-alkoxy-C₁-C₄-alkyl or C₃-C₈-cycloalkyl-C₁-C₄-alkyl;
R² is C₁-C₃-alkyl, C₁-C₃-haloalkyl or cyclopropylmethyl;
R³ is C₁-C₄-alkyl, C₁-C₄-haloalkyl, C₁-C₄-haloalkoxy or halogen;
n is 1 or 2.

8. The use of compounds of the formula I and their agriculturally useful salts as claimed in claim 1 for controlling phytopathogenic fungi.

9. A composition, which comprises a fungicidally effective amount of at least one compound of the formula I and/or at least one agriculturally useful salt of I as claimed in claim 1 and at least one carrier.

10. A method for controlling phytopathogenic fungi, which comprises treating the fungi or the materials, plants, seeds or the soil threatened by fungal attack with a fungicidally effective amount of at least one compound of the formula I as claimed in claim 1 and/or a salt of I.

## Revendications

1. Composés oxazin(thi)one de formule I 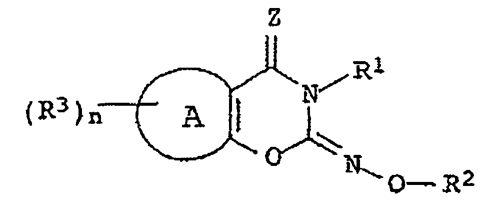 dans laquelle les variables Z, A, n, R¹, R² et R³ et n présentent les significations suivantes :
Z un oxygène ou un soufre ;
A un carbocycle à 5 ou 6 chaînons condensé avec le noyau oxazin(thi)one par l'intermédiaire de deux atomes de carbone, ou un hétérocycle à 5 ou 6 chaînons renfermant 1, 2 ou 3 hétéroatomes choisis parmi un azote, un oxygène et un soufre ;
n un nombre de 0 à 4 ;
R¹ un hydrogène, un alkyle en C₁-C₆, un alcényle en C₂-C₆, un alcynyle en C₂-C₆, qui sont chacun éventuellement substitués par O-R⁴, C(O)O-R⁵, NR⁶R⁷, C(O)NR⁶R⁷, S-R⁸, un cycloalkyle en C₃-C₈, un phényle ou par un hétérocyclyle saturé ou insaturé à 5 ou 6 chaînons, renfermant de 1 à 3 hétéroatomes choisis parmi un azote, un oxygène et un soufre où le phényle et l'hétérocyclyle sont éventuellement mono-, bi- ou tri- substitués, un halogénoalkyle en C₁-C₆, un halogénoalcényle en C₂-C₆, ou un halogénoalcynyle en C₂-C₆ ;
R² un alkyle en C₁-C₃, un halogénoalkyle en C₁-C₃ ou un cyclopropylméthyle ;
R³ un halogène, un cyano, un alkyle en C₁-C₆, un halogénoalkyle en C₁-C₆, un alcoxy en C₁-C₆ ou un halogénoalcoxy en C₁-C₆ ;
et où
R⁴, R⁵ représentent indépendamment l'un de l'autre un hydrogène, un alkyle en C₁-C₆, un alcényle en C₂-C₆, un alcynyle en C₂-C₆, un halogénoalkyle en C₁-C₆, un halogénoalcényle en C₂-C₆, un halogénoalcynyle en C₂-C₆, un hydroxy-C₁-C₄-alkyle, un hydroxycarbonyl-C₁-C₄-alkyle, un C₁-C₄-alcoxy-C₁-C₄-alkyle ou un C₁-C₄-alcoxycarbonyl-C₁-C₄-alkyle ;
R⁶, R⁷ représentent indépendamment l'un de l'autre, un alkyle en C₁-C₆, un alcoxy en C₁-C₆, un alcényle en C₂-C₆, un alcynyle en C₂-C₆, un halogénoalkyle en C₁-C₆, un halogénoalcoxy en C₁-C₆, un halogénoalcényle en C₂-C₆, un halogénoalcynyle en C₂-C₆, un hydroxy-C₁-C₄-alkyle, un hydroxycarbonyl-C₁-C₄-alkyle, un C₁-C₄-alcoxy-C₁-C₄-alkyle ou un C₁-C₄-alcoxycarbonyl-C₁-C₄-alkyle ;
R⁶ et R⁷, conjointement avec l'atome d'azote auquel ils sont liés peuvent également former un hétérocycle azoté saturé à 5, 6 ou 7 chaînons, qui peut renfermer un hétéroatome supplémentaire choisi parmi un oxygène, un azote et un soufre et/ou peut être substitué par de 1 à 4 groupes alkyle en C₁-C₆ ;
R⁸ représente un alkyle en C₁-C₆, un alcényle en C₂-C₆, un alcynyle en C₂-C₆, un halogénoalkyle en C₁-C₆, un halogénoalcényle en C₂-C₆, un halogénoalcynyle en C₂-C₆, un hydroxy-C₁-C₄-alkyle, un hydroxycarbonyl-C₁-C₄-alkyle, un C₁-C₄-alcoxy-C₁-C₄-alkyle ou un C₁-C₄-alcoxycarbonyl-C₁-C₄-alkyle ;
ainsi que les sels utilisables sur le plan agricole des composés oxazin(thi)one I.

2. Composés selon la revendication 1, dans lesquels A représente un noyau benzénique, pyridinique ou thiophénique condensé.

3. Composés selon la revendication 1 ou 2, dans lesquels R¹ représente un alkyle en C₁-C₆, un alcényle en C₂-C₆, un halogénoalkyle en C₁-C₆, un halogénoalcényle en C₂-C₆, un C₁-C₄-alcoxy-C₁-C₄-alkyle ou un C₃-C₈-cycloalkyl-C₁-C₄-alkyle.

4. Composés selon l'une quelconque des revendications précédentes, dans lesquels n vaut 1, 2, 3 ou 4.

5. Composés selon l'une quelconque des revendications précédentes, dans lesquels R³ représente un alkyle en C₁-C₄, un halogénoalkyle en C₁-C₄, un halogénoalcoxy en C₁-C₄ ou un halogène.

6. Composés selon l'une quelconque des revendications précédentes, dans lesquels A représente un noyau benzélique condensé et les variables Z, R¹, R², R³ et n présentent les significations suivantes :
Z un oxygène ;
R¹ un alkyle en C₁-C₆, un alcényle en C₂-C₆, un halogénoalkyle en C₁-C₆, un halogénoalcényle en C₂-C₆, un C₁-C₄-alcoxy-C₁-C₄-alkyle ou un C₃-C₈-cycloalkyl-C₁-C₄-alkyle ;
R² un alkyle en C₁-C₃, un halogénoalkyle en C₁-C₃ ou un cyclopropylméthyle ;
R³ un alkyle en C₁-C₄, un halogénoalkyle en C₁-C₄, un halogénoalcoxy en C₁-C₄ ou un halogène ;
n 1 ou 2 ;

7. Composés selon l'une quelconque des revendications 1 à 5, dans lesquels A représente un noyau thiophénique condensé et les variables Z, R¹, R², R³ et n présentent les significations suivantes :
Z un oxygène ;
R¹ un alkyle en C₁-C₆, un alcényle en C₂-C₆, un halogénoalkyle en C₁-C₆, un halogénoalcényle en C₂-C₆, un C₁-C₄-alcoxy-C₁-C₄-alkyle ou un C₃-C₈-cycloalkyl-C₁-C₄-alkyle ;
R² un alkyle en C₁-C₃, un halogénoalkyle en C₁-C₃ ou un cyclopropylméthyle ;
R³ un alkyle en C₁-C₄, un halogénoalkyle en C₁-C₄, un halogénoalcoxy en C₁-C₄ ou un halogène ;
n 1 ou 2 ;

8. Utilisation de composés de formule I et de leurs sels utilisables sur le plan agricole selon la revendication 1 pour la lutte contre des champignons phytopathogènes.

9. Composition comprenant une quantité à action fongicide d'au moins un composé de formule I et/ou d'au moins un sel utilisable sur le plan agricole de I selon la revendication 1 ainsi qu'au moins une matière de support.

10. Procédé pour la lutte contre des champignons phytopathogènes, dans lequel les champignons ou les matériaux, les plantes, les semences ou les sols menacés d'une infestation par des champignons sont traités avec une quantité à action fongicide d'au moins un composé de formule I selon la revendication 1 et/ou d'un sel de I.
